(19) Europäisches Patentamt
European Patent Office
Office européen des brevets

(11) Numéro de publication : **0 382 634 B1**

(12) # FASCICULE DE BREVET EUROPEEN

(45) Date de publication du fascicule du brevet : **04.05.94 Bulletin 94/18**

(21) Numéro de dépôt : **90400334.0**

(22) Date de dépôt : **07.02.90**

(51) Int. Cl.[5] : **C07D 237/20,** C07D 403/12, C07D 401/12, C07D 401/04, C07D 409/04, C07D 401/14, A61K 31/50

(54) **Nouveaux dérivés de la pyridazine, procédé de préparation et compositions pharmaceutiques en contenant.**

(30) Priorité : **07.02.89 FR 8901547**
**07.02.89 FR 8901548**

(43) Date de publication de la demande : **16.08.90 Bulletin 90/33**

(45) Mention de la délivrance du brevet : **04.05.94 Bulletin 94/18**

(84) Etats contractants désignés : **AT BE CH DE DK ES FR GB GR IT LI LU NL SE**

(56) Documents cités :
**EP-A- 0 072 726**
**EP-A- 0 117 171**
**FR-A- 2 141 697**
**US-A- 4 590 194**

(73) Titulaire : **ELF SANOFI**
**32-34, rue Marbeuf**
**F-75008 Paris (FR)**

(72) Inventeur : **Bourguignon, Jean-Jacques**
**2 rue du Feldwasser**
**F-67150 Hipsheim (FR)**
Inventeur : **Brodin, Roger**
**26, Mas des Bruyères, Avenue de Monsieur Teste**
**F-34070 Montpellier (FR)**
Inventeur : **Olliero, Dominique**
**12, Allée Jeanne Bourgeois Hameau de l'Aiguelongue**
**F-34070 Montpellier (FR)**
Inventeur : **Wermuth, Camille Georges**
**3, rue de la Côte d'Azur**
**F-67100 Strasbourg (FR)**
Inventeur : **Worms, Paul**
**10, rue Davois**
**F-34980 - St Gely du Fesc (FR)**

(74) Mandataire : **Gillard, Marie-Louise et al**
**Cabinet Beau de Loménie 158, rue de l'Université**
**F-75340 Paris Cédex 07 (FR)**

EP 0 382 634 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Depuis de nombreuses années, des dérivés de pyridazine ont été proposés en tant que médicaments.

Dans un grand nombre de cas, il s'agit de substances actives sur le système cardiovasculaire et présentant en particulier un effet hypotenseur ou vasodilateur, dans d'autres cas on a mentionné pour des dérivés de pyridazine une action anti-inflammatoire ou analgésique.

En particulier, les brevets français 2 141 697, 2 510 997 et 2 510 998 et les demandes de brevet européen EP-A-072 726 et EP-A-117 171 divulguent des dérivés de pyridazine diversement substitués sur le cycle pyridazinique et portant tous en position 3 un substituant aminé du type

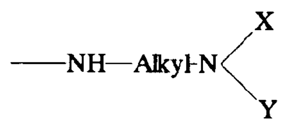

où X et Y représentent indépendamment l'hydrogène, un groupe alkyle ou forment avec l'atome d'azote auquel ils sont liés un hétérocycle, tel que la morpholine.

Tous ces composés présentent une activité sur le système nerveux central en tant qu'antidépresseurs.

Le brevet US 4 590 194 décrit lui des (3-amino-6-pyridinyl) pyridazines dont le groupe amino est mono- ou disubstitué par un groupe méthyle. Ces composés sont utiles comme agents cardiotoniques.

Selon la présente invention, on a maintenant trouvé qu'en modifiant la nature et/ou la position des substituants du cycle pyridazinique, on obtient des composés ayant perdu leur activé antidépressive et ayant acquis une activité intéressante en tant que ligands cholinergiques muscariniques de type $M_1$.

Selon un premier aspect, la présente invention a pour objet de nouveaux dérivés de pyridazine répondant à la formule générale :

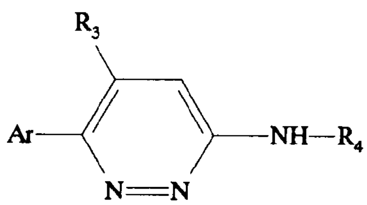

(I)

dans laquelle

- Ar représente un groupe de formule :

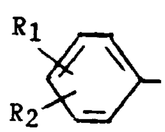

,

  un groupe pyridyle ou un groupe thiényle ;
- $R_1$ et $R_2$ désignent chacun indépendamment l'hydrogène, un atome d'halogène, un groupe hydroxyle, un groupe trifluorométhyle, un groupe alcoxy en $C_1$-$C_4$, un groupe alkyle en $C_1$-$C_4$ ;
- $R_3$ représente un groupe alkyle en $C_1$-$C_4$ ou un groupe phényle ;
- $R_4$ représente :
  - un groupe

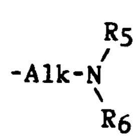

dans lequel Alk désigne un groupe alkylène en $C_1$-$C_6$, $R_5$ représente l'hydrogène ou un groupe alkyle

en $C_1$-$C_6$, $R_6$ représente un groupe alkyle en $C_1$-$C_4$, un benzyle, un cycloalkyle en $C_3$-$C_7$, ou encore $R_5$ et $R_6$ constituent avec l'atome d'azote auquel ils sont liés un hétérocycle choisi parmi la morpholine, la thiomorpholine, la pyrrolidine, la N-méthyl pipérazine, la pipéridine non substituée ou substituée par un ou plusieurs groupes méthyle, par un hydroxyle, par un phényle ou par un benzyle ;

- un groupe

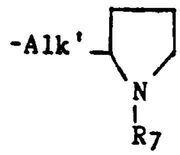

dans lequel Alk' désigne un groupe alkylène en $C_1$-$C_3$ et $R_7$ désigne un groupe alkyle en $C_1$-$C_4$ ;

- un groupe

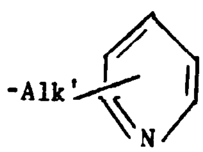

dans lequel Alk' est tel que défini ci-dessus et substitue la pyridine en position 2, 3, ou 4 ;

avec la limitation que $R_1$ et $R_2$ ne désignent pas simultanément l'hydrogène lorsque $R_4$ représente un groupe $(CH_2)_2$ $NR_6R_6$ ;

ainsi que les sels des composés de formule (I) avec les acides minéraux ou organiques.

Les composés (I) selon l'invention, dans lesquels Ar représente un groupe :

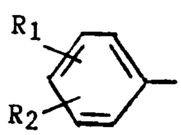

sont préférés.

Parmi ces composés, particulièrement préférés sont ceux dans lesquels $R_4$ représente l'un des groupes suivants :

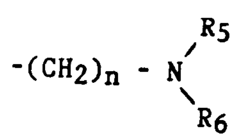

ou

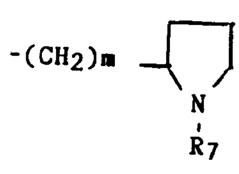

dans lesquels $R_6$, $R_6$ et $R_7$ ont les significations indiquées ci-dessus pour (I), n est compris entre 1 et 6 de préférence égal à 2, 3 ou 4 et m est compris entre 1 et 3, de préférence égal à 1 ou 2.

Lorsque le composé (I) présente un carbone asymétrique, les 2 stéréoisomères font partie de l'invention.

Selon un second aspect, la présente invention concerne un procédé de préparation des composés de formule (I) représenté par le schéma réactionnel suivant :

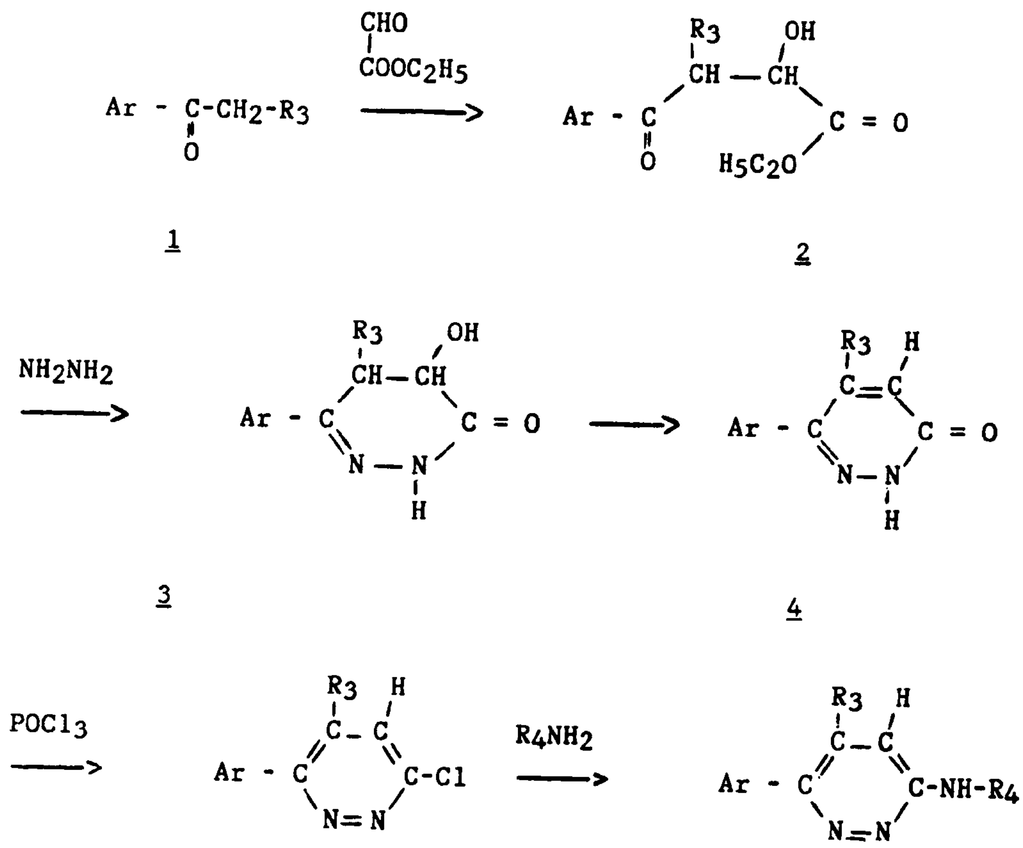


1

2

3

4

5

(I)

A partir de la cétone 1, par chauffage avec le glyoxylate d'éthyle à une température comprises entre 80 et 140°C, on obtient l'hydroxy céto ester 2. Le mélange réactionnel brut est alors repris dans un solvant inerte tel que le n-butanol et additionné d'hydrate d'hydrazine. Par chauffage au reflux pendant 24 heures, on obtient l'hydroxypyridazinone 3 qui chauffée en milieu acide conduit par déshydratation à la 2H-pyridazinone-3 4.

Cette dernière chauffée avec de l'oxychlorure de phosphore en excès fournit la chloro-3 pyridazine 5. On opère sans solvant ou en présence d'un solvant inerte tel que l'acétonitrile.

Enfin le dérivé chloré 5 chauffé à une température comprise entre 100 et 150°C avec un large excès d'amine $R_4NH_2$ en présence d'une petite quantité de chlorure d'ammonium conduit au composé (I). On opère sans solvant ou dans un solvant inerte comme le n-butanol. Le produit (I) est isolé par extraction et purifié par chromatographie.

Eventuellement, la base ainsi obtenue peut être salifiée selon un procédé connu et notamment par action d'une quantité équimoléculaire d'acide au sein d'un solvant convenable.

Les composés (I) dans lesquels Ar représente un groupe hydroxyphényle ou dihydroxyphényle, sont préparés à partir des composés (I) correspondants dans lesquels Ar représente un groupe méthoxyphényle ou diméthoxyphényle par déméthylation en milieu acide.

Les exemples suivants illustrent, sans la limiter, la préparation de composés de formule (I).

EXEMPLE 1

[(N-éthylpyrrolidinyl)-2 méthylamino]-3 méthyl-5 phényl-6 pyridazine (SR 96185)

(I) $R_1 = R_2 = H$ $R_3 = CH_3$

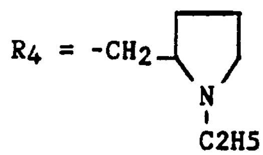

A) Chloro-3 méthyl-5 phényl-6 pyridazine

1. Hydroxy-2 méthyl-3 phényl-4 oxo-4 butyrate d'éthyle.

On chauffe à 135°C pendant 5 heures, le mélange de 13,4 g de propiophénone et 15,3 g de glyoxylate d'éthyle.

Le produit ainsi obtenu est utilisé tel quel pour l'opération suivante.

2. Méthyl-5 phényl-6 2H-pyridazinone-3.

On dissout le produit brut obtenu ci-dessus dans 150 ml de n-butanol, puis on ajoute 9,44 ml d'hydrate d'hydrazine et chauffe au reflux pendant 24 heures.

On distille à pression ordinaire une partie du n-butanol de façon à éliminer l'eau formée dans la réaction sous forme d'azéotrope. On concentre ensuite à siccité sous vide. On reprend le résidu dans un mélange de 100 ml d'acide acétique et 10 ml d'acide chlorhydrique concentré. On chauffe le mélange à 100°C pendant 4 heures. On verse la solution dans l'eau froide et laisse cristalliser.

On essore le solide et sèche.

Poids : 11,6g F : 218°C.

3. Chloro-3 méthyl-5 phényl-6 pyridazine.

A 12 g de pyridazinone obtenue ci-dessus on ajoute 50 ml d'oxychlorure de phosphore et chauffe à 80°C pendant 4 heures.

On verse lentement le mélange sur de la glace et alcalinise avec une solution de soude à 20 %.

On essore le précipité, lave abondamment avec de l'eau et recristallise dans l'isopropanol.

On obtient 9,9 g du produit attendu.

F : 122°C.

B) SR 96185

On chauffe à 130°C pendant 3 heures sous atmosphère inerte, le mélange de 2 g du dérivé chloré obtenu ci-dessus, 5 g d'aminométhyl-2 éthyl-1 pyrrolidine et 0,5 g de chlorure d'ammonium. On verse le mélange réactionnel dans l'eau et extrait avec de l'acétate d'éthyle. On sèche la solution et évapore le solvant à siccité. On chromatographie le résidu sur une colonne de silice. En éluant avec le mélange acétate d'éthyle-méthanol 95-5 (vol/vol) on obtient une huile qui cristallise dans une petite quantité d'acétate d'éthyle.

Poids : 2 g F : 92°C.

EXEMPLE 2

[(Diéthylamino-2 éthyl) amino]-3 méthyl-5 (méthoxy-4 phényl)-6 pyridazine, dioxalate (SR 96194 A)
(I) $R_1$ = $-OCH_3(4)$, $R_2$ = H, $R_3$ = $CH_3$,

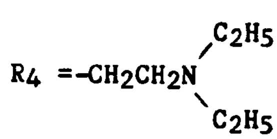

A) Chloro-3 méthyl-5 (méthoxy-4 phényl)-6 pyridazine.

1. Hydroxy-2 méthyl-3 (méthoxy-4 phényl)-4 oxo-4 butyrate d'éthyle.

On chauffe à 135°C pendant 15 heures, le mélange de 82 g de méthoxy-4 propiophénone et 76,6 g de glyoxylate d'éthyle.

Le produit ainsi obtenu est utilisé tel quel pour l'opération suivante.

2. Méthyl-5 (méthoxy-4 phényl)-6 2H-pyridazinone-3.

On dissout le produit brut obtenu ci-dessus dans 250 ml de n-butanol, puis on ajoute 38 g d'hydrate

d'hydrazine et chauffe au reflux pendant 24 heures.

On distille à pression ordinaire une partie du n-butanol de façon à éliminer l'eau formée dans la réaction sous forme d'azéotrope. On concentre ensuite à siccité sous vide. On reprend le résidu dans un mélange de 450 ml d'acide acétique et 50 ml d'acide chlorhydrique concentré. On chauffe le mélange à 100°C pendant 4 heures. On verse la solution dans l'eau froide et laisse cristalliser.

On essore le solide et sèche.

Poids : 40 g F :216°C.

3. Chloro-3 méthyl-5 (méthoxy-4 phényl)-6 pyridazine.

A 10 g de pyridazinone obtenue ci-dessus on ajoute 250 ml d'oxychlorure de phosphore et chauffe à 80°C pendant 10 heures.

On verse lentement le mélange sur de la glace et alcalinise avec une solution de soude à 20 %.

On essore le précipité, lave abondamment avec de l'eau et recristallise dans le méthanol.

On obtient 9 g du produit attendu.

F : 132°C.

B) SR 96194 A

On chauffe à 135°C pendant 4 heures sous atmosphère inerte, le mélange de 2,3 g du dérivé chloré obtenu ci-dessus, 5 g de N-N diéthyléthylène diamine et 0,5 g de chlorure d'ammonium. On verse le mélange réactionnel dans l'eau et extrait avec de l'acétate d'éthyle. On sèche la solution et évapore le solvant à siccité.

Poids : 2,3 g

<u>Dioxalate</u>

On dissout la base ci-dessus dans l'isopropanol à chaud et ajoute 2,2 équivalents d'acide oxalique dissous dans le minimum d'isopropanol bouillant. Par refroidissement le dioxalate précipite. On l'essore et recristallise dans l'éthanol absolu.

F : 136°C.

EXEMPLES 3 A 80

A) En opérant comme indiqué dans l'exemple 1A, mais en faisant varier la cétone de départ, on obtient les chloro-3 pyridazines réunies dans les tableaux 1 et 2 suivants :

TABLEAU 1

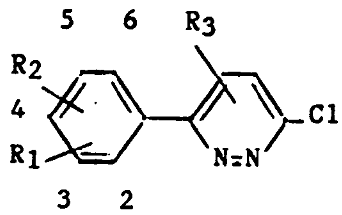

| $R_1$ | $R_2$ | $R_3$ | Constantes physiques |
|---|---|---|---|
| Cl (4) | H | $-CH_3$ | F : 178-180°C |
| Cl (4) | H | $-CH_2CH_3$ | F : 86°C |
| Cl (2) | Cl (4) | $-CH_3$ | F : 116°C |
| Cl (3) | H | $-CH_3$ | F : 95°C |
| $CF_3$ (3) | H | $-CH_3$ | F : 120°C |
| F (4) | H | $-CH_3$ | F : 153°C |
| $CH_3$ (4) | H | $-CH_3$ | F : 165-166°C |
| Cl (4) | H | $-CH_2CH_2CH_3$ | F : 95°C |
| $OCH_3$ (4) | H | $-CH_2CH_2CH_3$ | F : 68-69°C |
| H | H | $-CH_3$ | F : 123-124°C |
| H | H | phényl | F : 115°C |

| | | | |
|---|---|---|---|
| Cl (3) | Cl (5) | $-CH_3$ | F : 104°C |
| Cl (3) | Cl (4) | $-CH_3$ | F : 167°C |
| H | H | $-CH_2CH_2CH_3$ | F : 60°C |
| $OCH_3(2)$ | H | $-CH_3$ | F : 74°C |

TABLEAU 2

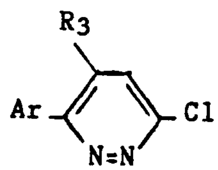

| Ar | $R_3$ | Constantes physiques |
|---|---|---|
| Pyridyl-2 | $-CH_3$ | 135°C |
| Pyridyl-3 | $-CH_3$ | 142°C |
| Thiényl-2 | $-CH_3$ | 99°C |

B) A partir des dérivés chlorés des exemples 1A, 2A et 3A et en faisant varier les amines utilisées, on obtient suivant la technique de l'exemple 1B les composés (I) rassemblés dans les tableaux 3 et 4. Ces composés sont caractérisés par leur point de fusion (F) ou leur pouvoir rotatoire $[\alpha]_D$

TABLEAU 3

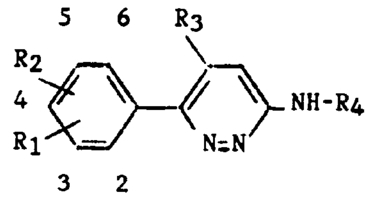

| Ex n° | N° Réf SR | $R_1$ | $R_2$ | $R_3$ | $R_4$ | Sel ou base F ou $[\alpha]_D$ |
|---|---|---|---|---|---|---|
| 3 | 96181 | H | H | $-(CH_2)_2-CH_3$ | $-CH_2-$ 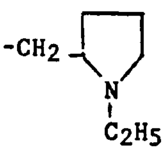 | Base 94°C |
| 4 | 96198 | Cl(4) | H | $-CH_3$ | " " | Base 90°C |
| 5 | 96222 | Cl(2) | Cl(4) | $-CH_3$ | " " | Base 113°C |
| 6 | 46004A | $CF_3(3)$ | H | $-CH_3$ | " " | Dichlorhydrate 0,5 $H_2O$ 180°C (décomposition) |
| 7 | 96204A | H | H | $-CH_3$ | $-CH_2-$ 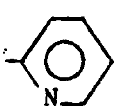 | Dichlorhydrate 221°C |

| | | | | | | |
|---|---|---|---|---|---|---|
| 8 | 96240A | Cl(4) | H | $-CH_3$ | " " | Dichlorhydrate 222-223°C |
| 9 | 46005A | Cl(3) | H | $-CH_3$ | " " | Chlorhydrate, $1H_2O$ 182°C |
| 10 | 45991A | Cl(4) | H | $-CH_2CH_3$ | " " | Dichlorhydrate 215-217°C |
| 11 | 96220A | H | H | $-CH_3$ | $-CH_2-$ 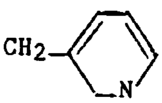 | Dichlorhydrate $1H_2O$ 189°C |
| 12 | 96205A | H | H | $-CH_3$ | $-(CH_2)_2-$ 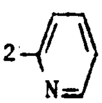 | Dichlorhydrate $1H_2O$ 133°C |
| 13 | 96239A | Cl(4) | H | $-CH_3$ | " " | Dichlorhydrate 224°C |
| 14 | 46035A | Cl(3) | H | $-CH_3$ | $-CH_2-$ 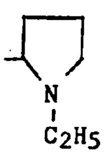 | Dichlorhydrate $1H_2O$ 172°C |
| 15 | 46079A | Cl(4) | H | $-CH_2CH_3$ | " " | Fumarate 163-165°C |

| | | | | | | |
|---|---|---|---|---|---|---|
| 16 | 96193A | $-OCH_3(4)$ | H | $-CH_3$ | $-CH_2CH_2-$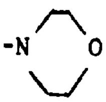 | Dichlorhydrate 219°C |
| 17 | 96197A | Cl(4) | H | $-CH_3$ | " " | Dichlorhydrate, 0,5$H_2O$ 247°C |
| 18 | 96223A | Cl(2) | Cl(4) | $-CH_3$ | " " | Dichlorhydrate 250°C (décomposition) |
| 19 | 45964A | $CF_3(3)$ | H | $-CH_3$ | " " | Dichlorhydrate, 2$H_2O$ 183°C |
| 20 | 45944A | Cl(3) | H | $-CH_3$ | " " | Dichlorhydrate 1,5$H_2O$ 212°C |
| 21 | 46179A | Cl(3) | Cl(5) | $-CH_3$ | " " | Dichlorhydrate, 1$H_2O$ 198°C |
| 22 | 46197A | Cl(4) | H | $-CH_2-CH_3$ | $-CH_2-CH_2-$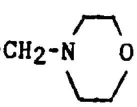 | Difumarate 168-170°C |
| 23 | 46222A | F(4) | H | $-CH_3$ | " " | Dichlorhydrate 217-219°C |
| 24 | 46223A | $-CH_3(4)$ | H | $-CH_3$ | " " | Fumarate 165-167°C |

| | | | | | | |
|---|---|---|---|---|---|---|
| 25 | 46224A | Cl(3) | Cl(4) | $-CH_3$ | " " | Dichlorhydrate 228-230°C |
| 26 | 46405A | Cl(4) | H | $-CH_3$ | $-CH_2-CH_2-N(iPr)_2$ | Dichlorhydrate, $1H_2O$ 157-160°C |
| 27 | 46534A | Cl(4) | H | $-n\ C_3H_7$ | " " | Fumarate 0,5 $H_2O$ 130°C |
| 28 | 46431A | Cl(4) | H | $-CH_3$ | $-CH_2-CH_2-$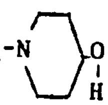 | Dichlorhydrate 226-228°C |
| 29 | 46432A | Cl(4) | H | $-CH_3$ | $-CH_2-CH_2-$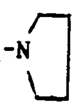 | Dichlorhydrate $0,5H_2O$ 208-210°C |
| 30 | 46514A | Cl(4) | H | $-CH_3$ | $-CH_2-CH_2-$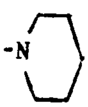 | Dichlorhydrate $0,5H_2O$ 172-174°C |
| 31 | 46637A | Cl(4) | H | $-n.C_3H_7$ | " " | Dichlorhydrate $1H_2O$ 205°C |
| 32 | 46636A | H | H | $-n.C_3H_7$ | " " | Dichlorhydrate $1H_2O$ 207°C |

| | | | | | | |
|---|---|---|---|---|---|---|
| 33 | 96224A | Cl(4) | H | $-CH_3$ | $/C_2H_5$<br>$-CH_2CH_2-N$<br>$\backslash C_2H_5$ | Dioxalate<br>131°C |
| 34 | 96230A | Cl(2) | Cl(4) | $-CH_3$ | " " | Dioxalate<br>$1H_2O$ 106°C |
| 35 | 96194A | -OCH3(4) | H | $-CH_3$ | " " | Dioxalate<br>136°C |
| 36 | 96266A | Cl(3) | H | $-CH_3$ | " " | Dioxalate<br>136°C |
| 37 | 96232A | Cl(4) | H | $-CH_3$ | $/CH3$<br>$-CH_2CH_2N$<br>$\backslash CH_3$ | Dichlorhy-<br>drate<br>258-259°C |
| 38 | 46010A | Cl(4) | H | $-CH_2CH_3$ | " " | Dichlorhy-<br>drate<br>$0,3H_2O$<br>223-225°C |
| 39 | 46081A | F(4) | H | $-CH_3$ | " " | Dichlorhy-<br>drate<br>$0,25H_2O$<br>233-235°C |
| 40 | 46082A | $-CH_3(4)$ | H | $-CH_3$ | " " | Dichlorhy-<br>drate<br>235-237°C |
| 41 | 45960A | Cl(4) | H | $-CH_3$ | $/CH_3$<br>$-(CH_2)_3-N$<br>$\backslash CH_3$ | Dichlorhy-<br>drate<br>256-258°C |

| | | | | | | |
|---|---|---|---|---|---|---|
| 42 | 46080A | Cl(4) | H | $-CH_2CH_3$ | " " | Dichlorhydrate $0,5H_2O$: 202°C |
| 43 | 46731A | Cl(4) | H | $-CH_3$ | 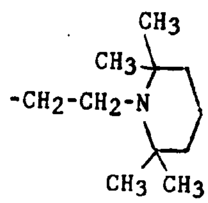 | Fumarate 213-215°C |
| 44 | 46730A | H | H | $-n.C_3H_7$ | " " | 2,5 Fumarate: 193-195°C |
| 45 | 96231A | Cl(4) | H | $-CH_3$ | $-CH_2CH_2NH-CH_3$ | Oxalate, $1H_2O$ 111-113°C |
| 46 | 45961A | Cl(4) | H | $-CH_3$ | 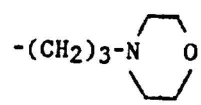 | Dichlorhydrate 250-252°C |
| 47 | 45988A | Cl(4) | H | $-CH_3$ | 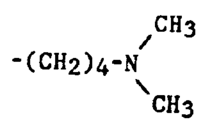 | Dichlorhydrate 244-246°C |
| 48 | 45989A | Cl(4) | H | $-CH_2-CH_3$ | " " | Dichlorhydrate $2H_2O$ 214-216°C |
| 49 | 46377A | H | H | $-n.C_3H_7$ | 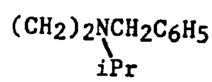 | Dichlorhydrate $0,5H_2O$ 182-184°C |

| | | | | | | |
|---|---|---|---|---|---|---|
| 50 | 46532A | H | H | $-n.C_3H_7$ | $-CH_2-CH_2-$ 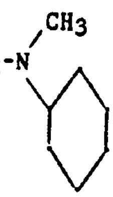 | Fumarate,<br>$1H_2O$<br>152°C |
| 51 | 46728A | Cl(4) | H | $-CH_3$ | $(CH_2)_2-$<br>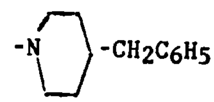 | Dichlorhydrate $0,5H_2O$:<br>224-226°C |
| 52 | 96290A | H | H | $-CH_3$ | $-CH_2-CH_2-$ 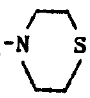 | Dioxalate<br>181°C |
| 53 | 96291 | H | H | $-CH_3$ | $(CH_2)_2-$<br>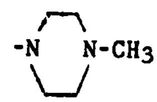 | Base 98°C |
| 54 | 46352A | H | H | $-n.C_3H_7$ | $-CH_2-CH_2-N(iPr)_2$ | Fumarate<br>156-157°C |
| 55 | 46359A | H | H | " | $-(CH_2)_2-NCH_2CH_3$<br>$(CH_2)_3CH_3$ | Fumarate<br>113°-114°C |
| 56 | 46378A | H | H | " | $-(CH_2)_2N(nBu)_2$ | Fumarate<br>95-96°C |
| 57 | 46533A | H | H | " | $-(CH_2)_2-N(nPr)_2$ | Fumarate<br>109-110°C |
| 58 | 46180 | Cl(3) | Cl(5) | $-CH_3$ | $-CH_2-$ 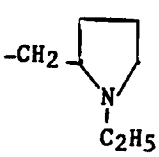 | Dichlorhydrate<br>192°C |

| | | | | | | |
|---|---|---|---|---|---|---|
| 59 | 46195 | Cl(4) | H | $-C_2H_5$ | $-CH_2-$ 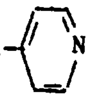 | Fumarate 180-182°C |
| 60 | 46196 | $CH_3(4)$ | H | $-CH_3$ | $-CH_2-$ 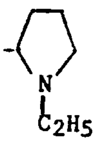 | Base 106°C |
| 61 | 96268 | Cl(4) | H | $-CH_3$ | (+) $-CH_2-$ 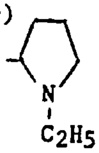 | Huile jaune $\alpha^{19}=+65°$ (2% $CHCl_3$) |
| 62 | 96269 | Cl(4) | H | $-CH_3$ | (-) $-CH_2-$ 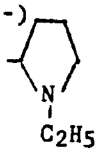 | Huile jaune $\alpha^{19}=-68°$ (2% CHC13) |
| 63 | 96272 | H | H | $-nC_3H_7$ | $-CH_2-$ 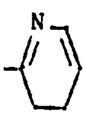 | Dichlorhydrate 218°C |
| 64 | 46433 | H | H | $-nC_3H_7$ | (+) $-CH_2-$ 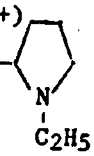 | Base 83°C $\alpha^{22}=+68,6°$ (C=1, $CHCl_3$) |
| 65 | 46434 | H | H | $-nC_3H_7$ | (-) $-CH_2-$ 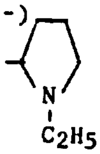 | Base 82°C $\alpha^{21}=-70°$ (C=1, $CHCl_3$) |

| | | | | | | |
|---|---|---|---|---|---|---|
| 66 | 46430 | F(4) | H | $-CH_3$ | $-CH_2$ 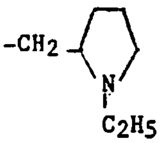 | Dichlorhydrate 206°C |
| 67 | 46514 | Cl(4) | H | $-CH_3$ | $-CH_2-CH_2-$ 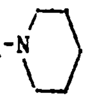 | Dichlorhydrate $1H_2O$ 174°C |
| 68 | 46636 | H | H | $-nC_3H_7$ | $-CH_2-CH_2-$ 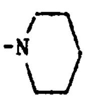 | Dichlorhydrate $1H_2O$ 207°C |
| 69 | 46637 | Cl(4) | H | $-nC_3H_7$ | $-CH_2-CH_2-$ 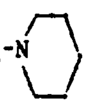 | Dichlorhydrate $1H_2O$ 205°C |
| 70 | 47046 | H | H | $-C_6H_5$ | $-CH_2-$ 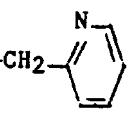 | Hémifumarate 165°C |
| 71 | 47186 | H | H | $-C_6H_5$ | $-CH_2-$ 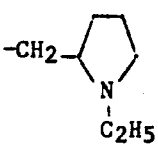 | Fumarate 158°C |
| 72 | 47226 | H | H | $-C_6H_5$ | $-CH_2-CH_2-$ 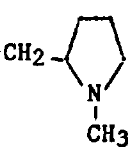 | Sesquifumarate 191°C |

| | | | | | | |
|---|---|---|---|---|---|---|
| 73 | 96305 | OH(2) | H | $-CH_3$ | $-CH_2$ 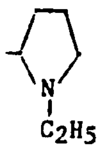 | Base, 0,3$H_2O$ 160°C |
| 74 | 96306A | Cl(4) | H | $-CH_3$ | $-CH_2CH_2N$ 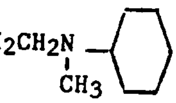 | Dichlorhydrate 0,5$H_2O$ 257°C |
| 75 | 96307A | Cl(4) | H | $-CH_3$ | $-CH_2CH_2N$ 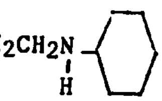 | Dioxalate 0,5$H_2O$ 179°C |
| 76 | 96308A | H | H | $-CH_3$ | $-CH_2CH_2N$ 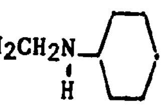 | Chlorhydrate $H_2O$ 196°C |

TABLEAU 4

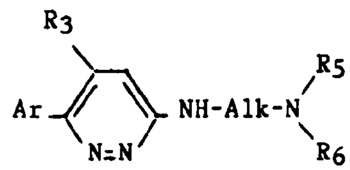

| Ex | N° Réf SR | Ar | $R_3$ | 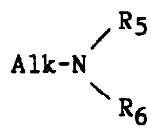 | Sel ou base F |
|---|---|---|---|---|---|
| 77 | 46457A | Pyridyl-2 | $-CH_3$ | $-CH_2-CH_2-N\ (iPr)_2$ | Fumarate 145°C |
| 78 | 46578A | Pyridyl-3 | $-CH_3$ | " " | Fumarate 1,5$H_2O$ 175°C |
| 79 | 46640A | Pyridyl-2 | $-CH_3$ | $-CH_2-CH_2-N$ 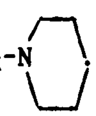 | Difumarate 146°C |
| 80 | 46641A | Thiényl-2 | $-CH_3$ | " " | Dichlorhydrate 0,5$H_2O$ 265°C |

Les produits selon l'invention ont été étudiés en ce qui concerne leurs propriétés pharmacologiques et en particulier en ce qui concerne leur affinité pour les récepteurs cholinergiques muscariniques.

In vitro, les composés (I) ont été essayés suivant la technique décrite par Watson J.D. et al. (Life Science, 1982, 31, 2019-2029) en ce qui concerne leur activité sur les récepteurs de type M1 et selon la technique décrite par Hammer R. et al. (Nature, 1980, 283, 90,92) et Hulme E.C. et al. (Molecular Pharmacology, 1978, 14, 737-750), en ce qui concerne leur activité sur les récepteurs de type M2.

Les composés selon l'invention présentent une bonne affinité sur les récepteurs de type M1 et une spécificité marquée pour les récepteurs centraux de type M1 vis-à-vis des récepteurs périphériques de type M2.

A titre d'exemple, le composé SR 96181 a montré une concentration inhibitrice 50 exprimée en micromole de 0,03 sur le ligand des récepteurs M1 et de 0,35 sur le ligand des récepteurs M2.

De même, le composé SR 96204 A a montré des concentrations inhibitrices 50 de 0,75 et 50 respectivement sur le ligand des récepteurs M1 et M2.

In vivo, les composés selon l'invention ont été essayés sur le test des rotations induites par la pirenzépine décrit par Worms P. et al. (Psychopharmacology, 1987, 93, 489-493) modifié en ce que l'administration des produits par voie orale qui a eu lieu 4 heures au lieu de 30 minutes avant l'injection de pirenzépine.

A la dose de 3 mg par kg de poids corporel, les produits selon l'invention inhibent fortement le nombre des

rotations induites par la pirenzépine.

Ainsi à titre d'exemple, les composés SR 96181 et SR 96204 A inhibent respectivement de 71 % et 67 % les rotations induites par la pirenzépine.

Par ailleurs, on trouvera dans le tableau 5, les résultats obtenus avec divers composés de formule (I).

On a fait figurer dans ce tableau, les résultats obtenus avec le seul composé du brevet français n° 2 510 998 substitué par un groupe alkyle en position 5 et décrit dans ce brevet en tant qu'antidépresseur.

Ce composé répond à la formule :

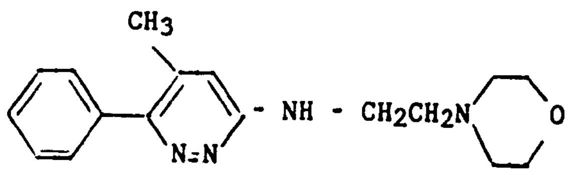

Composé A

TABLEAU 5

| Produits n° | % inhibition rotations pirenzépine à la dose de 3 mg/kg per os |
|---|---|
| SR 96197 A | - 70 % ** |
| SR 96224 A | - 59 % ** |
| SR 96231 A | - 40 % ** |
| SR 96232 A | - 40 % ** |
| SR 96223 A | - 74 % ** |
| SR 96230 A | - 41 % ** |
| SR 45964 A | - 38 % ** |
| Composé A | - 20 % * |

Test t de Student

* $p < 0{,}05$

** $p < 0{,}01$

A la dose de 3 mg/kg per os, les composés selon l'invention inhibent le nombre des rotations induites par la pirenzépine avec une intensité 2 à 3,5 fois plus grande que le composé de l'art antérieur.

Enfin les composés selon l'invention n'ont mis en évidence aucun signe de toxicité aux doses où ils sont actifs.

Par suite les composés (I) peuvent être utilisés en tant que médicaments.

Les résultats indiqués permettent d'envisager l'utilisation des produits selon l'invention dans tous les cas où se manifeste un déficit cholinergique et notamment pour le traitement des troubles mnésiques, cognitifs, des syndromes dégénératifs liés à la sénescence et aux démences séniles.

Selon un autre de ses aspects la présente invention concerne donc les compositions pharmaceutiques contenant au moins un des composés de formule (I) ou un de leurs sels pharmaceutiquement acceptables en tant qu'ingrédient actif.

Dans les compositions pharmaceutiques de la présente invention pour l'administration orale, sublinguale, transdermique ou rectale, les ingrédients actifs de formule (I) ci-dessus peuvent être administrés sous forme unitaire d'administration, en mélange avec les supports pharmaceutiques classiques, aux êtres humains notamment pour le traitement des démences séniles. Les formes unitaires d'administration appropriées comprennent les formes par voie orale, telles que les comprimés, les gélules, les poudres, les granulés et les solutions ou suspensions orales, les formes d'administration sublinguale et buccale, les formes d'administration sous-cutanée, intramusculaire ou intraveineuse et les formes d'administration rectale.

Afin d'obtenir l'effet désiré, la dose de principe actif peut varier entre 20 et 500 mg par jour.

Chaque dose unitaire peur contenir de 5 à 200 mg d'ingrédient actif en combinaison avec un support pharmaceutique. Cette dose unitaire peut être administrée 1 à 4 fois par jour.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut le traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans les gélules molles ou dures.

Les poudres ou les granulés dispersibles dans l'eau peuvent contenir l'ingrédient actif en mélange avec des agents de dispersion ou des agents mouillants, ou des agents de mise en suspension comme la polyvinylpyrrolidone, de même qu'avec des édulcorants ou des correcteurs de goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol et le butylèneglycol.

Le principe actif peut être formulé également sous forme de microcapsules, éventuellement avec un ou plusieurs supports ou additifs.

A titre de préparations galéniques on peut préparer des gélules :

EXEMPLE 81

| | |
|---|---|
| SR 96204 A | 0,010 g |
| Lactose | 0,050 g. |
| Stéarate de magnésium | 0,005 g. |

On mélange intimement les ingrédients ci-dessus et on verse le mélange dans des gélules de gélatine dure.

EXEMPLE 82

| | |
|---|---|
| SR 96197A | 0,010 g. |
| Lactose | 0,050 g. |
| Stéarate de magnésium | 0,005 g. |

## Revendications

### Revendications pour les Etats contractants suivants : AT, BE, CH, DE, DK, FR, GB, IT, LI, LU, NL, SE

**1.** Dérivés de la pyridazine de formule :

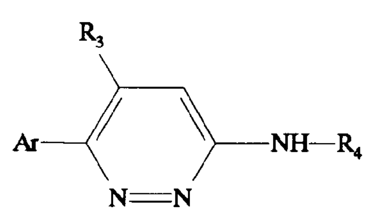

(I)

dans laquelle

- Ar représente un groupe :

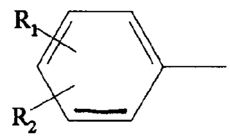
,

un pyridyle ou un thiényle ;

- $R_1$ et $R_2$ désignent chacun indépendamment l'hydrogène, un atome d'halogène, un groupe hydroxyle, un groupe trifluorométhyle, un groupe alcoxy en $C_1$-$C_4$, un groupe alkyle en $C_1$-$C_4$ ;
- $R_3$ représente un groupe alkyle en $C_1$-$C_4$ ou un phényle ;
- $R_4$ représente :
- un groupe

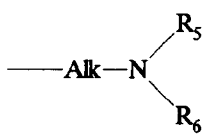

dans lequel Alk désigne un groupe alkylène en $C_1$-$C_6$, $R_5$ représente l'hydrogène ou un groupe alkyle en $C_1$-$C_6$, $R_6$ représente un groupe alkyle en $C_1$-$C_4$, un benzyle, un cycloalkyle en $C_3$-$C_7$, ou encore $R_5$ et $R_5$ constituent avec l'atome d'azote auquel ils sont liés un hétérocycle choisi parmi la morpholine, la thiomorpholine, la pyrrolidine, la N-méthyl pipérazine, la pipéridine non substituée ou substituée par un ou plusieurs groupes méthyle, par un hydroxyle, par un phényle ou par un benzyle ;

- un groupe

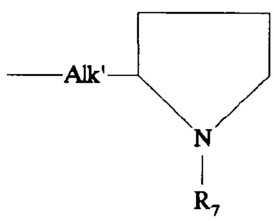

dans lequel Alk' désigne un groupe alkylène en $C_1$-$C_3$ et $R_7$ désigne un groupe alkyle en $C_1$-$C_4$ ;

- un groupe

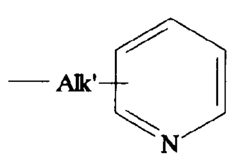

dans lequel Alk' est tel que défini ci-dessus et substitue la pyridine en position 2, 3 ou 4 ;
avec la limitation que $R_1$ et $R_2$ ne désignent par simultanément l'hydrogène lorsque $R_4$ représente un groupe $(CH_2)_2NR_5R_5$ ; ainsi que leurs sels avec les acides minéraux ou organiques.

**2.** Composé selon la revendication 1 dans lequel Ar représente un groupe :

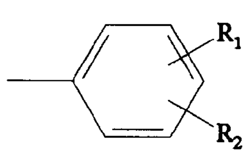

dans lequel $R_1$ et $R_2$ ont les mêmes significations que dans la revendication 1.

**3.** Composé selon l'une des revendications 1 ou 2, répondant à la formule :

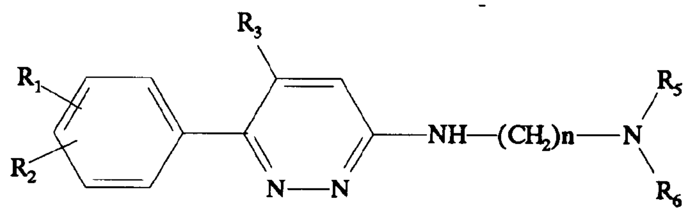

dans laquelle $R_1$, $R_2$, $R_3$, $R_5$ et $R_6$ ont les mêmes significations que dans la revendication 1 et n compris entre 1 et 6, avec la limitation que $R_1$ et $R_2$ ne sont pas simultanément l'hydrogène lorsque n = 2.

4. Composé selon la revendication 3, caractérisé en ce que n est égal à 2, 3 ou 4.

5. Composé selon l'une des revendications 1 ou 2 répondant à la formule :

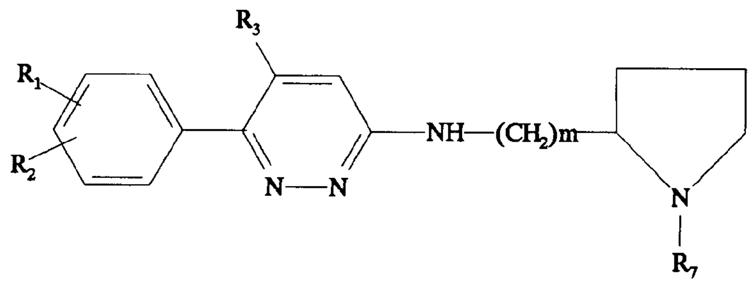

dans laquelle $R_1$, $R_2$, $R_3$ et $R_7$ ont les mêmes significations que dans la revendication 1 et m est un nombre entier compris entre 1 et 3.

6. Composé selon la revendication 5, caractérisé en ce m est égal à 1 ou 2.

7. Procédé de préparation des composés de formule (I) selon la revendication 1, caractérisé en ce que :
   - on chauffe une cétone convenablement substituée de formule :

$$Ar\text{-}COCH_2\text{-}R_3 \qquad \underline{1}$$

     avec du glyoxylate d'éthyle pour former l'hydroxyester de formule :

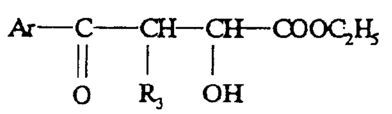

2

   - on chauffe l'ester 2 avec de l'hydrate d'hydrazine pour former l'hydroxy pyridazinone de formule :

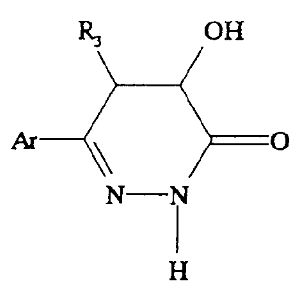

3

   - on déshydrate par chauffage en milieu acide le produit ainsi obtenu pour former la 2H pyridazinone-3 de formule :

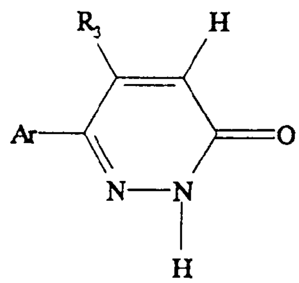

4

- on traite le produit ainsi obtenu par un excès d'oxychlorure de phosphore à chaud pour former la chloro-3 pyridazine de formule :

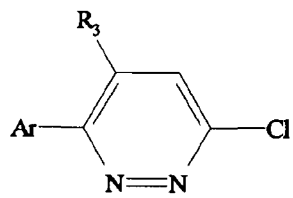

5

- on chauffe le dérivé chloré 5 avec un excès d'amine $R_4NH_2$ en présence éventuellement de chlorure d'ammonium pour obtenir le composé (I) ;
- éventuellement on salifie le composé (I) avec un acide minéral ou organique selon un procédé connu.

**8.** Procédé selon la revendication 7, caractérisé en ce que l'on utilise une cétone 1 dans laquelle Ar représente un groupe :

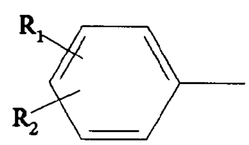

dans lequel $R_1$ et $R_2$ sont tels que définis dans la revendication 1.

**9.** Procédé selon la revendication 7, caractérisé en ce qu'on utilise une amine $R_4NH_2$ dans laquelle $R_4$ représente l'un des groupes suivants :

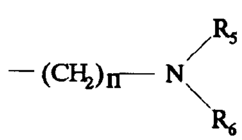

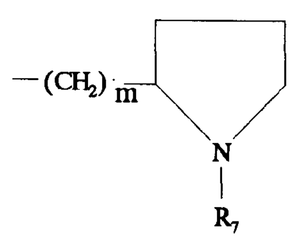

dans lesquels $R_5$, $R_5$ et $R_7$ ont les significations indiquées dans la revendication 1 et n est compris entre 1 et 6, de préférence égal à 2, 3 ou 4 et m est compris entre 1 et 3, de préférence égal à 1 ou 2.

**10.** Compostions pharmaceutiques contenant à titre de principe actif un composé de formule (I) ou l'un de

ses sels pharmaceutiquement acceptables en combinaison avec un véhicule pharmaceutiquement acceptable.

**11.** Compositions pharmaceutiques selon la revendication 10, caractérisées en ce qu'elles se présentent sous forme d'unitéS de dosage contenant 5 à 200 mg de principe actif.

**Revendications pour l'Etat contractant suivant : GR**

**1.** Dérivés de la pyridazine de formule :

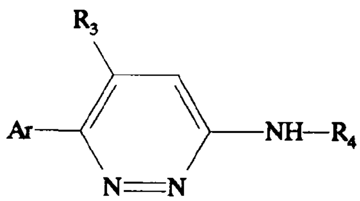

(I)

dans laquelle

- Ar représente un groupe :

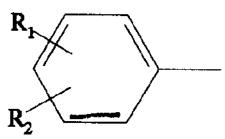

,

un pyridyle ou un thiényle ;

- $R_1$ et $R_2$ désignent chacun indépendamment l'hydrogène, un atome d'halogène, un groupe hydroxyle, un groupe trifluorométhyle, un groupe alcoxy en $C_1$-$C_4$, un groupe alkyle en $C_1$-$C_4$ ;
- $R_3$ représente un groupe alkyle en $C_1$-$C_4$ ou un phényle ;
- $R_4$ représente :
- un groupe

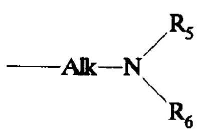

dans lequel Alk désigne un groupe alkylène en $C_1$-$C_6$, $R_5$ représente l'hydrogène ou un groupe alkyle en $C_1$-$C_6$, $R_6$ représente un groupe alkyle en $C_1$-$C_4$, un benzyle, un cycloalkyle en $C_3$-$C_7$, ou encore $R_5$ et $R_5$ constituent avec l'atome d'azote auquel ils sont liés un hétérocycle choisi parmi la morpholine, la thiomorpholine, la pyrrolidine, la N-méthyl pipérazine, la pipéridine non substituée ou substituée par un ou plusieurs groupes méthyle, par un hydroxyle, par un phényle ou par un benzyle ;
- un groupe

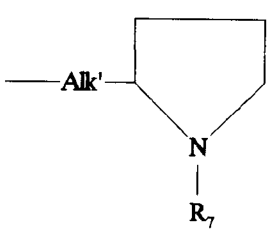

dans lequel Alk' désigne un groupe alkylène en $C_1$-$C_3$ et $R_7$ désigne un groupe alkyle en $C_1$-$C_4$ ;
- un groupe

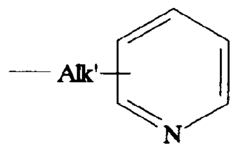

dans lequel Alk' est tel que défini ci-dessus et substitue la pyridine en position 2, 3 ou 4 ; avec la limitation que $R_1$ et $R_2$ ne désignent par simultanément l'hydrogène lorsque $R_4$ représente un groupe $(CH_2)_2NR_5R_5$ ; ainsi que leurs sels avec les acides minéraux ou organiques.

2. Composé selon la revendication 1 dans lequel Ar représente un groupe :

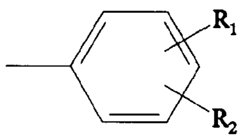

dans lequel $R_1$ et $R_2$ ont les mêmes significations que dans la revendication 1.

3. Composé selon l'une des revendications 1 ou 2, répondant à la formule :

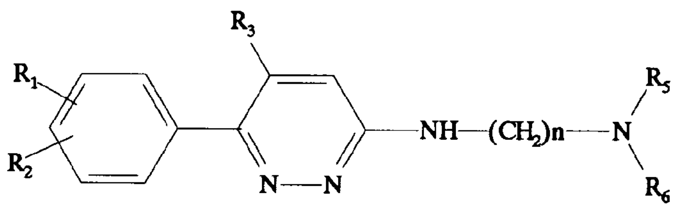

dans laquelle $R_1$, $R_2$, $R_3$, $R_5$ et $R_5$ ont les mêmes significations que dans la revendication 1 et n compris entre 1 et 6, avec la limitation que $R_1$ et $R_2$ ne sont pas simultanément l'hydrogène lorsque n = 2.

4. Composé selon la revendication 3, caractérisé en ce que n est égal à 2, 3 ou 4.

5. Composé selon l'une des revendications 1 ou 2 répondant à la formule :

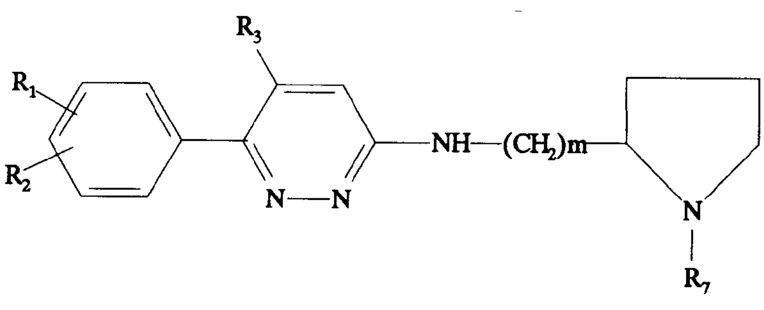

dans laquelle $R_1$, $R_2$, $R_3$ et $R_7$ ont les mêmes significations que dans la revendication 1 et m est un nombre entier compris entre 1 et 3.

6. Composé selon la revendication 5, caractérisé en ce m est égal à 1 ou 2.

7. Procédé de préparation des composés de formule (I) selon la revendication 1, caractérisé en ce que :
   - on chauffe une cétone convenablement substituée de formule :

$$Ar\text{-}COCH_2\text{-}R_3 \qquad \underline{1}$$

   avec du glyoxylate d'éthyle pour former l'hydroxyester de formule :

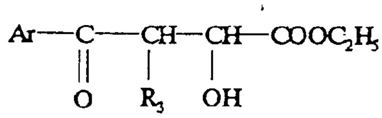

2

- on chauffe l'ester 2 avec de l'hydrate d'hydrazine pour former l'hydroxy pyridazinone de formule :

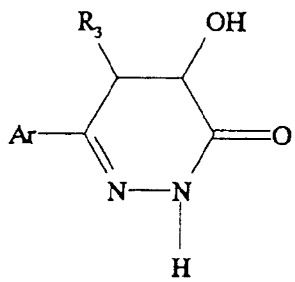

3

- on déshydrate par chauffage en milieu acide le produit ainsi obtenu pour former la 2H pyridazinone-3 de formule :

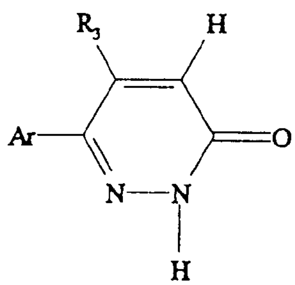

4

- on traite le produit ainsi obtenu par un excès d'oxychlorure de phosphore à chaud pour former la chloro-3 pyridazine de formule :

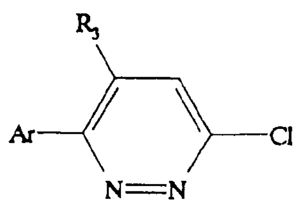

5

- on chauffe le dérivé chloré 5 avec un excès d'amine $R_4NH_2$ en présence éventuellement de chlorure d'ammonium pour obtenir le composé (I);
- éventuellement on salifie le composé (I) avec un acide minéral ou organique selon un procédé connu.

**8.** Procédé selon la revendication 7, caractérisé en ce que l'on utilise une cétone 1 dans laquelle Ar représente un groupe :

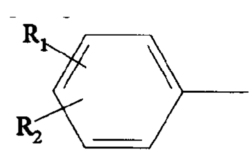

dans lequel $R_1$ et $R_2$ sont tels que définis dans la revendication 1.

**9.** Procédé selon la revendication 7, caractérisé en ce qu'on utilise une amine $R_4NH_2$ dans laquelle $R_4$ représente l'un des groupes suivants :

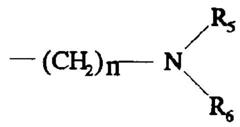

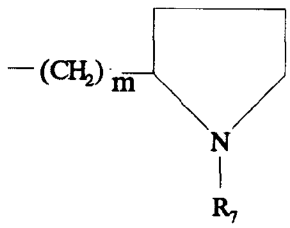

dans lesquels $R_5$, $R_6$ et $R_7$ ont les significations indiquées dans la revendication 1 et n est compris entre 1 et 6, de préférence égal à 2, 3 ou 4 et m est compris entre 1 et 3, de préférence égal à 1 ou 2.

**10.** Procédé pour l'obtention de compositions pharmaceutiques utiles notamment pour le traitement du déficit cholinergique caractérisé en ce qu'il consiste à utiliser comme principe actif un composé de formule (I) tel que défini dans l'une quelconque des revendications 1 à 6.

**11.** Procédé selon la revendication 10, caractérisé en ce que l'on utilise 5 à 200 mg de principe actif pour former des compositions sous forme d'unités de dosage.

**Revendications pour l'Etat contractant suivant : ES**

**1.** Procédé pour l'obtention de dérivés de la pyridazine de formule :

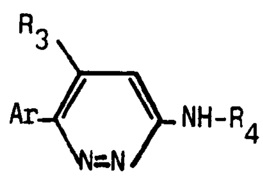

dans laquelle

- Ar représente un groupe :

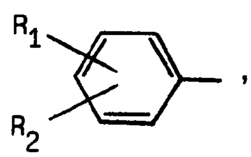

un pyridyle ou un thiényle ;

- $R_1$ et $R_2$ désignent chacun indépendamment l'hydrogène, un atome d'halogène, un groupe hydroxyle, un groupe trifluorométhyle, un groupe alcoxy en $C_1$-$C_4$, un groupe alkyle en $C_1$-$C_4$ ;
- $R_3$ représente un groupe alkyle en $C_1$-$C_4$ ou un phényle ;
- $R_4$ représente ;
- un groupe

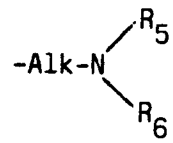

dans lequel Alk désigne un groupe alkylène en $C_1$-$C_6$, $R_5$ représente l'hydrogène ou un groupe alkyle en $C_1$-$C_6$, $R_6$ représente un groupe alkyle en $C_1$-$C_4$, un benzyle, un cycloalkyle en $C_3$-$C_7$, ou encore $R_5$ et $R_5$ constituent avec l'atome d'azote auquel ils sont liés un hétérocycle choisi parmi la morpholine, la thiomorpholine, la pyrrolidine, la N-méthyl pipérazine, la pipéridine non substituée ou substituée par un ou plusieurs groupes méthyle, par un hydroxyle, par un phényle ou par un benzyle ;

- un groupe

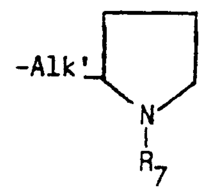

dans lequel Alk' désigne un groupe alkylène en $C_1$-$C_3$ et $R_7$ désigne un groupe alkyle en $C_1$-$C_4$ ;

- un groupe

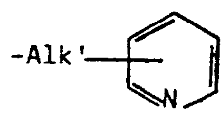

dans lequel Alk' est tel que défini ci-dessus et substitue la pyridine en position 2, 3 ou 4 ;
avec la limitation que $R_1$ et $R_2$ ne désignent pas simultanément l'hydrogène lorsque $R_4$ représente un groupe $(CH_2)_2NR_5R_5$ ;
ainsi que leurs sels avec les acides minéraux ou organiques ; caractérisé en ce qu'il consiste :

- à chauffer une cétone convenablement substituée de formule :

$Ar$-$COCH_2$-$R_3$ 1

avec du glyoxylate d'éthyle pour former l'hydroxyester de formule :

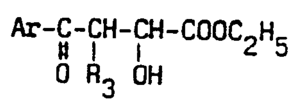

2

- à chauffer l'ester 2 avec de l'hydrate d'hydrazine pour former l'hydroxy pyridazinone de formule :

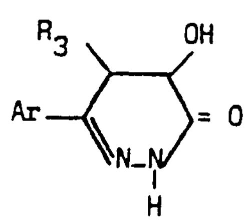

3

- à déshydrater par chauffage en milieu acide le produit ainsi obtenu pour former la 2H pyridazinone-3 de formule :

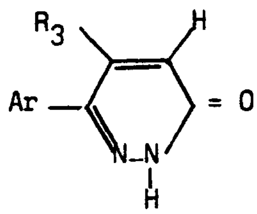

4

- à traiter le produit ainsi obtenu par un excès d'oxychlorure de phosphore à chaud pour former la chloro-3 pyridazine de formule :

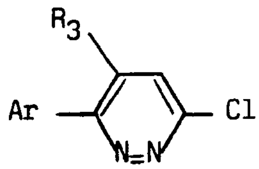

5

- à chauffer le dérivé chloré 5 avec un excès d'amine $R_4NH_2$ en présence éventuellement de chlorure d'ammonium pour obtenir le composé (I) ;
- éventuellement à salifier le composé (I) avec un acide minéral ou organique selon un procédé connu.

2. Procédé selon la revendication 1, caractérisé en ce que l'on utilise une cétone 1 dans laquelle Ar représente un groupe :

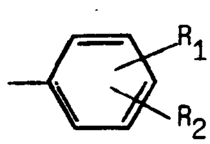

dans lequel $R_1$ et $R_2$ sont tels que définis dans la revendication 1.

3. Procédé selon la revendication 1, caractérisé en ce que l'on utilise une amine $R_4NH_2$ dans laquelle $R_4$ représente l'un des groupes suivants :

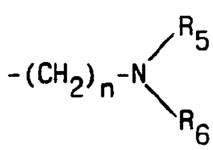

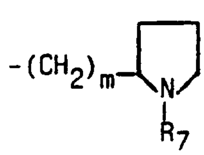

dans laquelle $R_5$, $R_6$ et $R_7$ ont les significations indiquées dans la revendication 1 et n est compris entre 1 et 6, de préférence égal à 2, 3 ou 4 et m est compris entre 1 et 3, de préférence égal à 1 ou 2.

4. Procédé pour l'obtention de compositions pharmaceutiques utiles notamment pour le traitement du déficit cholinergique caractérisé en ce qu'il consiste à utiliser comme principe actif un composé de formule (I) tel que défini dans l'une quelconque des revendications 1 à 3.

5. Procédé selon la revendication 4, caractérisé en ce que l'on utilise 5 à 200 mg de principe actif pour former des compositions sous forme d'unité de dosage.

## Patentansprüche

### Patentansprüche für folgende Vertragsstaaten : AT, BE, CH, DE, DK, FR, GB, IT, LI, LU, NL, SE

1. Pyridazinderivate der Formel

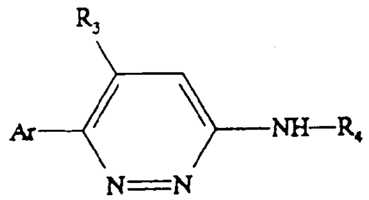

(I),

in der bedeuten:

- Ar eine Gruppe der Formel

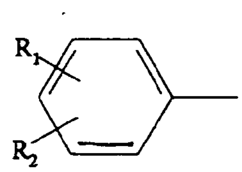

,

Pyridyl, Thienyl,
worin $R_1$ und $R_2$ unabhängig Wasserstoff, ein Halogenatom, Hydroxy, Trifluormethyl, $C_1$-$_4$-Alkoxy oder $C_1$-$_4$-Alkyl darstellen;

- $R_3$ $C_1$-$_4$-Alkyl oder Phenyl,
- $R_4$
- eine Gruppe der Formel

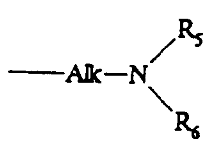

,

worin Alk $C_{1-6}$-Alkylen, $R_5$ Wasserstoff oder $C_{1-6}$-Alkyl und $R_5$ $C_{1-4}$-Alkyl, Benzyl oder $C_{3-7}$-Cycloalkyl darstellen, oder worin $R_5$ und $R_6$ mit dem Stickstoffatom, an das sie gebunden sind, einen Heterocyclus bilden, der ausgewählt ist unter Morpholin, Thiomorpholin, Pyrrolidin, N-Methylpiperazin, und Piperidin, das gegebenenfalls mit einer oder mehreren Methylgruppen, einer Hydroxygruppe, einer Phenylgruppe oder einer Benzylgruppe substituiert ist,

- eine Gruppe

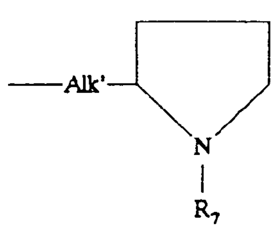

,

worin Alk' $C_{1-3}$-Alkylen und $R_7$ $C_{1-4}$-Alkyl darstellen,
oder
- eine Gruppe

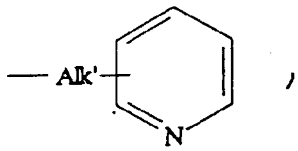

worin Alk' dasselbe wie oben bedeutet und sich in 2-, 3- oder 4-Stellung des Pyridins befindet, mit der Einschränkung, daß $R_1$ und $R_2$ nicht gleichzeitig Wasserstoff bedeuten, wenn $R_4$ eine Gruppe $(CH_2)_2NR_5R_5$ darstellt,
sowie ihre Salze mit den anorganischen und organischen Säuren.

2. Verbindungen nach Anspruch 1, wobei Ar eine Gruppe der Formel

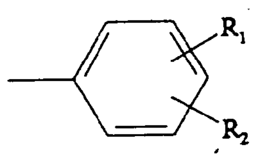

bedeutet,
worin $R_1$ und $R_2$ die gleiche Bedeutung wie in Anspruch 1 haben.

3. Verbindungen nach einem der Ansprüche 1 und 2, die der Formel

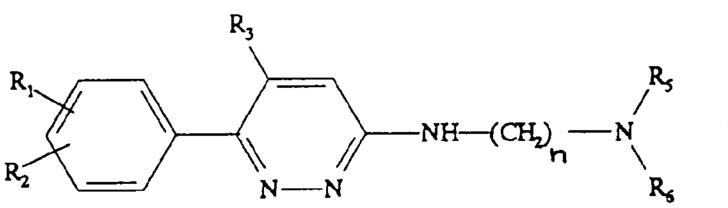

entspricht,
worin $R_1$, $R_2$, $R_3$, $R_5$ und $R_5$ die in Anspruch 1 angegebene Bedeutung haben und n eine Zahl von 1 bis 6 ist mit der Einschränkung, daß bei n = 2 $R_1$ und $R_2$ nicht gleichzeitig Wasserstoff sind.

4. Verbindungen nach Anspruch 3, dadurch gekennzeichnet, daß n gleich 2, 3 oder 4 ist.

5. Verbindungen nach einem der Ansprüche 1 und 2 entsprechend der Formel

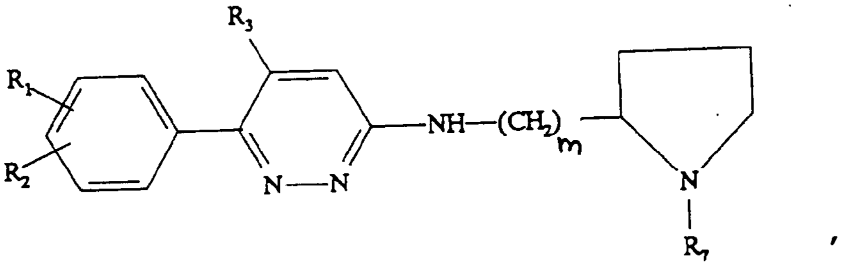

worin $R_1$, $R_2$, $R_3$ und $R_7$ die gleiche Bedeutung wie in Anspruch 1 haben und m eine ganze Zahl von 1 bis 3 bedeutet.

**6.** Verbindungen nach Anspruch 5,
dadurch gekennzeichnet, daß
m 1 oder 2 bedeutet.

**7.** Verfahren zur Herstellung von Verbindungen der Formel (I) nach Anspruch 1,
gekennzeichnet durch folgende Schritte:

- Erwärmen eines geeignet substituierten Ketons der Formel

  $Ar\text{-}COCH_2\text{-}R_3$ 1

  mit Ethylglyoxylat, unter Bildung des Hydroxyesters der Formel:

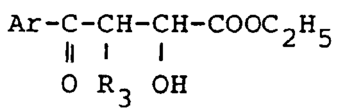

2,

- Erwärmen des Esters 2 mit Hydrazinhydrat zur Erzeugung des Hydroxypyridazinons der Formel

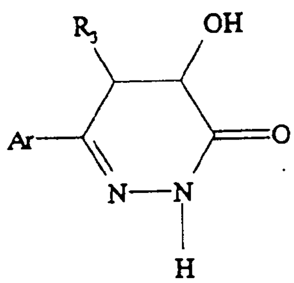

3,

- Wasserabspaltung bei dem so erhaltenen Produkt durch Erhitzen in saurem Medium unter Erhalt des 2H-Pyridazin-3-ons der Formel

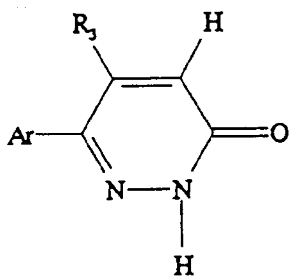

4,

- Behandlung des so erhaltenen Produktes mit überschüssigem Phosphoroxichlorid in der Wärme unter Bildung des 3-Chlorpyridazins der Formel

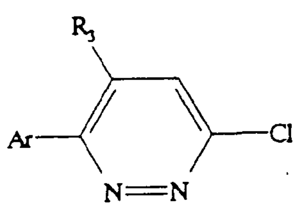

5

und

- Erwärmen des chlorierten Derivats 5 mit einem Überschuß des Amins $R_4NH_2$, gegebenenfalls in Gegenwart von Ammoniumchlorid, unter Erhalt der Verbindung (I)

- sowie gegebenenfalls Überführung der Verbindung (I) mit einer anorganischen oder einer organischen Säure nach einem bekannten Verfahren in ein entsprechendes Salz.

**8.** Verfahren nach Anspruch 7,
dadurch gekennzeichnet, daß
ein Keton 1 verwendet wird, in dessen Formel Ar eine Gruppe

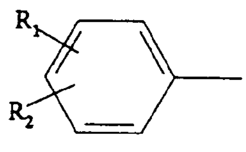

darstellt,
wobei $R_1$ und $R_2$ wie in Anspruch 1 definiert sind.

**9.** Verfahren nach Anspruch 7,
dadurch gekennzeichnet, daß
ein Amin $R_4NH_2$ verwendet wird, bei dem $R_4$ eine der folgenden Gruppen darstellt:

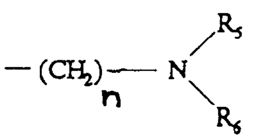

,

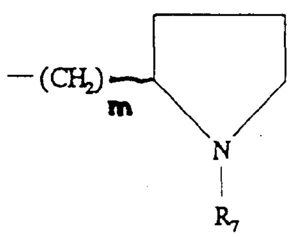

wobei $R_5$, $R_6$ und $R_7$ die in Anspruch 1 angegebenen Bedeutungen haben und n eine Zahl von 1 bis 6 und vorzugsweise 2, 3 oder 4 und m eine Zahl von 1 bis 3 und vorzugsweise 1 oder 2 bedeuten.

**10.** Pharmazeutische Zusammensetzungen, die als Wirkstoff eine Verbindung der Formel (I) oder eines ihrer pharmazeutisch akzeptablen Salze in Verbindung mit einem pharmazeutisch akzeptablen Träger enthält.

**11.** Pharmazeutische Zusammensetzungen nach Anspruch 10, dadurch gekennzeichnet, daß
sie in Form von Dosiereinheiten mit 5 bis 200 mg des Wirkstoffs eingesetzt werden.

**Patentanprüche für folgenden Vertragsstaat : GR**

**1.** Pyridazinderivate der Formel

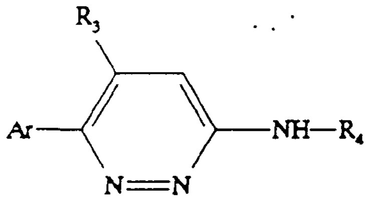

(I) ,

in der bedeuten:

- Ar eine Gruppe der Formel

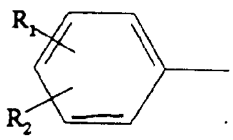

,

Pyridyl, Thienyl,
worin $R_1$ und $R_2$ unabhängig Wasserstoff, ein Halogenatom, Hydroxy, Trifluormethyl, $C_{1-4}$-Alkoxy oder $C_{1-4}$-Alkyl darstellen;

- $R_3$ $C_{1-4}$-Alkyl oder Phenyl,
- $R_4$
- eine Gruppe der Formel

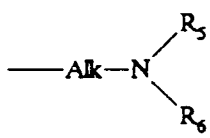

worin Alk $C_{1-6}$-Alkylen, $R_5$ Wasserstoff oder $C_{1-6}$-Alkyl und $R_5$ $C_{1-4}$-Alkyl, Benzyl oder $C_{3-7}$-Cycloalkyl darstellen, oder worin $R_5$ und $R_6$ mit dem Stickstoffatom, an das sie gebunden sind, einen Heterocyclus bilden, der ausgewählt ist unter Morpholin, Thiomorpholin, Pyrrolidin, N-Methylpiperazin, und Piperidin, das gegebenenfalls mit einer oder mehreren Methylgruppen, einer Hydroxygruppe, einer Phenylgruppe oder einer Benzylgruppe substituiert ist,

- eine Gruppe

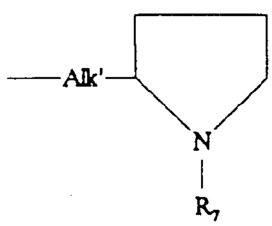

worin Alk' $C_{1-3}$-Alkylen und $R_7$ $C_{1-4}$-Alkyl darstellen,
oder

- eine Gruppe

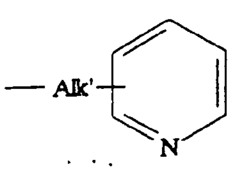

worin Alk' dasselbe wie oben bedeutet und sich in 2-, 3- oder 4-Stellung des Pyridins befindet, mit der Einschränkung, daß $R_1$ und $R_2$ nicht gleichzeitig Wasserstoff bedeuten, wenn $R_4$ eine Gruppe $(CH_2)_2NR_5R_5$ darstellt,

sowie ihre Salze mit anorganischen und organischen Säuren.

**2.** Verbindungen nach Anspruch 1, wobei Ar eine Gruppe der Formel

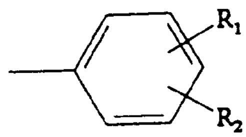

bedeutet,
worin $R_1$ und $R_2$ die gleiche Bedeutung wie in Anspruch 1 haben.

**3.** Verbindungen nach einem der Ansprüche 1 und 2,
die der Formel

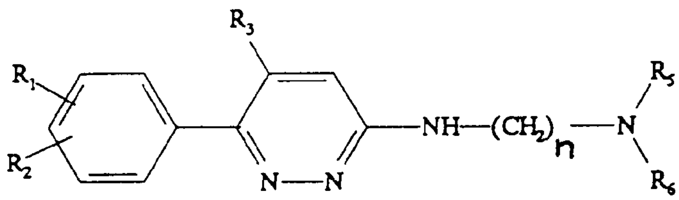

entspricht,
worin $R_1$, $R_2$, $R_3$, $R_5$ und $R_5$ die in Anspruch 1 angegebene Bedeutung haben und n eine Zahl von 1 bis 6 ist mit der Einschränkung, daß bei n = 2 $R_1$ und $R_2$ nicht gleichzeitig Wasserstoff sind.

**4.** Verbindungen nach Anspruch 3,
dadurch gekennzeichnet, daß
n gleich 2, 3 oder 4 ist.

**5.** Verbindungen nach einem der Ansprüche 1 und 2 entsprechend der Formel

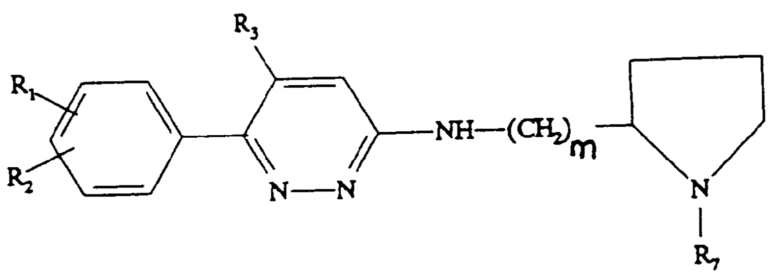

,

worin $R_1$, $R_2$, $R_3$ und $R_7$ die gleiche Bedeutung wie in Anspruch 1 haben und m eine ganze Zahl von 1 bis 3 bedeutet.

**6.** Verbindung nach Anspruch 5,
dadurch gekennzeichnet, daß
m 1 oder 2 bedeutet.

**7.** Verfahren zur Herstellung von Verbindungen der Formel (I) nach Anspruch 1,
gekennzeichnet durch folgende Schritte:

- Erwärmen eines geeignet substituierten Ketons der Formel

$$Ar\text{-}COCH_2\text{-}R_3 \quad \underline{1}$$

mit Ethylglyoxylat, unter Bildung des Hydroxyesters der Formel:

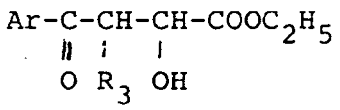

2,

- Erwärmen des Esters 2 mit Hydrazinhydrat zur Erzeugung des Hydroxypyridazinons der Formel

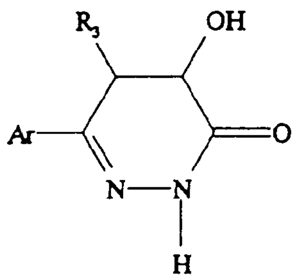

3,

- Wasserabspaltung bei dem so erhaltenen Produkt durch Erhitzen in saurem Medium unter Erhalt des 2H-Pyridazin-3-ons der Formel

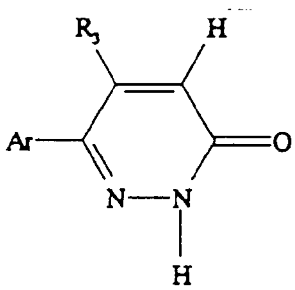

4,

- Behandlung des so erhaltenen Produktes mit überschüssigem Phosphoroxichlorid in der Wärme unter Bildung des 3-Chlorpyridazins der Formel

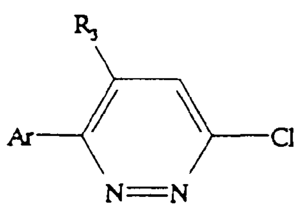

5

und

- Erwärmen des chlorierten Derivats 5 mit einem Überschuß des Amins $R_4NH_2$, gegebenenfalls in Gegenwart von Ammoniumchlorid, unter Erhalt der Verbindung (I)
- sowie gegebenenfalls Überführung der Verbindung (I) mit einer anorganischen oder einer organischen Säure nach einem bekannten Verfahren in ein entsprechendes Salz.

**8.** Verfahren nach Anspruch 7,
dadurch gekennzeichnet, daß
ein Keton 1 verwendet wird, in dessen Formel Ar eine Gruppe

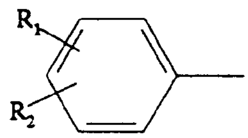

darstellt,
wobei $R_1$ und $R_2$ wie in Anspruch 1 definiert sind.

9. Verfahren nach Anspruch 7,
dadurch gekennzeichnet, daß
ein Amin $R_4NH_2$ verwendet wird, bei dem $R_4$ eine der folgenden Gruppen darstellt:

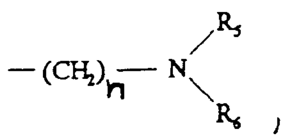

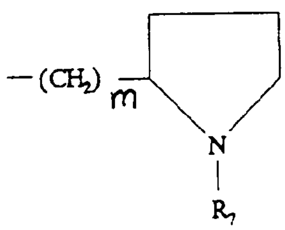

wobei $R_5$, $R_6$ und $R_7$ die in Anspruch 1 angegebenen Bedeutungen haben und n eine Zahl von 1 bis 6 und vorzugsweise 2, 3 oder 4 und m eine Zahl von 1 bis 3 und vorzugsweise 1 oder 2 bedeuten.

10. Verfahren zur Herstellung von pharmazeutischen Zusammensetzungen, die insbesondere zur Behandlung cholinerger Mangelzustände geeignet sind,
dadurch gekennzeichnet, daß
sie als Wirkstoff eine Verbindung der Formel (I) nach einem der Ansprüche 1 bis 6 enthalten.

11. Verfahren nach Anspruch 10,
dadurch gekennzeichnet, daß
5 bis 200 mg Wirkstoff zur Herstellung von Zusammensetzungen in Form von Dosiereinheiten eingesetzt werden.

**Patentansprüche für folgenden Vertragsstaat : ES**

1. Verfahren zur Herstellung von Pyridazinderivaten der Formel

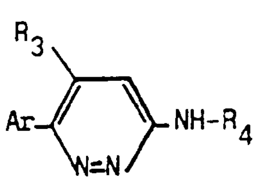

(I),

in der bedeuten:
- Ar eine Gruppe der Formel

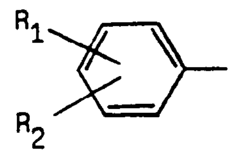
,

Pyridyl oder Thienyl,
worin $R_1$ und $R_2$ unabhängig Wasserstoff, ein Halogenatom, Hydroxy, Trifluormethyl, $C_{1-4}$-Alkoxy oder $C_{1-4}$-Alkyl darstellen;

- $R_3$ $C_{1-4}$-Alkyl oder Phenyl,
- $R_4$
- eine Gruppe der Formel

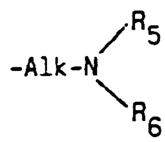
,

worin Alk $C_{1-6}$-Alkylen, $R_5$ Wasserstoff oder $C_{1-6}$-Alkyl und $R_5$ $C_{1-4}$-Alkyl, Benzyl oder $C_{3-7}$-Cycloalkyl darstellen, oder worin $R_5$ und $R_6$ mit dem Stickstoffatom, an das sie gebunden sind, einen Heterocyclus bilden, der ausgewählt ist unter Morpholin, Thiomorpholin, Pyrrolidin, N-Methylpiperazin und Piperidin, das gegebenenfalls mit einer oder mehreren Methylgruppen, einer Hydroxygruppe, einer Phenylgruppe oder einer Benzylgruppe substituiert ist,

- eine Gruppe

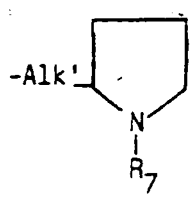
,

worin Alk' $C_{1-3}$-Alkylen und $R_7$ $C_{1-4}$-Alkyl darstellen,
oder

- eine Gruppe

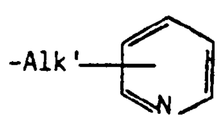
,

worin Alk' dasselbe wie oben bedeutet und sich in 2-, 3- oder 4-Stellung des Pyridins befindet,
mit der Einschränkung, daß $R_1$ und $R_2$ nicht gleichzeitig Wasserstoff bedeuten, wenn $R_4$ eine Gruppe $(CH_2)_2NR_5R_5$ darstellt,

sowie ihre Salze mit anorganischen und organischen Säuren,
**gekennzeichnet** durch folgende Schritte:

- Erwärmen eines geeignet substituierten Ketons der Formel

$$Ar\text{-}COCH_2\text{-}R_3 \qquad \underline{1}$$

mit Ethylglyoxylat, unter Bildung des Hydroxyesters der Formel:

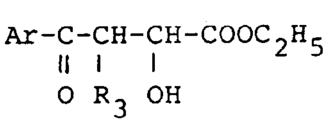
2,

- Erwärmen des Esters 2 mit Hydrazinhydrat zur Erzeugung des Hydroxypyridazinons der Formel

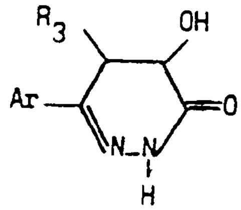

3

- Wasserabspaltung bei dem so erhaltenen Produkt durch Erhitzen in saurem Medium unter Erhalt des 2H-Pyridazin-3-ons der Formel

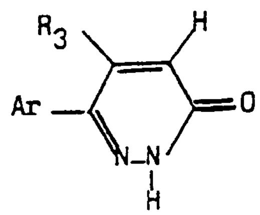

4,

- Behandlung des so erhaltenen Produktes mit überschüssigem Phosphoroxidchlorid in der Wärme unter Bildung des 3-Chlorpyridazins der Formel

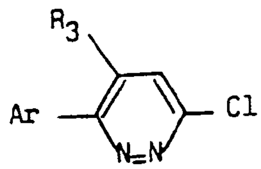

5

und

- Erwärmen des chlorierten Derivats 5 mit einem Überschuß des Amins $R_4NH_2$, gegebenenfalls in Gegenwart von Ammoniumchlorid, unter Erhalt der Verbindung (I)
- sowie gegebenenfalls Überführung der Verbindung (I) mit einer anorganischen oder einer organischen Säure nach einem bekannten Verfahren in ein entsprechendes Salz.

**2.** Verfahren nach Anspruch 1,
dadurch gekennzeichnet, daß
ein Keton 1 verwendet wird, in dessen Formel Ar eine Gruppe

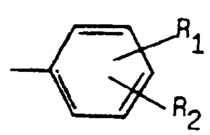

darstellt,
wobei $R_1$ und $R_2$ wie in Anspruch 1 definiert sind.

**3.** Verfahren nach Anspruch 1,
dadurch gekennzeichnet, daß
ein Amin $R_4NH_2$ verwendet wird, bei dem $R_4$ eine der folgenden Gruppen darstellt:

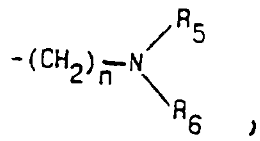

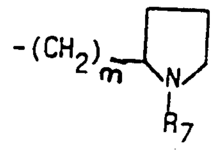

wobei $R_5$, $R_6$ und $R_7$ die in Anspruch 1 angegebenen Bedeutungen haben und n eine Zahl von 1 bis 6 und vorzugsweise 2, 3 oder 4 und m eine Zahl von 1 bis 3 und vorzugsweise 1 oder 2 bedeuten.

4. Verfahren zur Herstellung von pharmazeutischen Zusammensetzungen, die insbesondere zur Behandlung cholinerger Mangelzustände geeignet sind,
dadurch gekennzeichnet, daß
sie als Wirkstoff eine Verbindung der Formel (I) nach einem der Ansprüche 1 bis 3 enthalten.

5. Verfahren nach Anspruch 4,
dadurch gekennzeichnet, daß
5 bis 200 mg Wirkstoff zur Herstellung von Zusammensetzungen in Form von Dosiereinheiten eingesetzt werden.

## Claims

### Claims for the following Contracting States :AT, BE, CH, DE, DK, FR, GB, IT, LI, LU, NL, SE

1. Pyridazine derivatives of the formula:

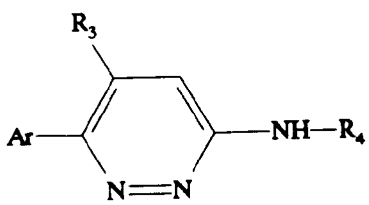 (I)

in which
- Ar is a group

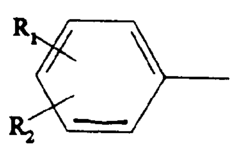 ,

a pyridyl or a thienyl;
- $R_1$ and $R_2$ independently are each hydrogen, a halogen atom, a hydroxyl group, a trifluoromethyl group, a $C_1$-$C_4$ alkoxy group or a $C_1$-$C_4$ alkyl group;
- $R_3$ is a $C_1$-$C_4$ alkyl group or a phenyl;
- $R_4$ is
- a group

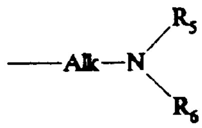

in which Alk is a $C_1$-$C_6$ alkylene group, $R_5$ is hydrogen or a $C_1$-$C_6$ alkyl group and $R_5$ is a $C_1$-$C_4$ alkyl group, a benzyl or a $C_3$-$C_7$ cycloalkyl, or else $R_5$ and $R_6$ form, with the nitrogen atom to which they are bonded, a heterocycle selected from morpholine, thiomorpholine, pyrrolidine, N-methylpiperazine and piperidine which is unsubstituted or substituted by one or more methyl groups, by a hydroxy, by a phenyl or by a benzyl;

- a group

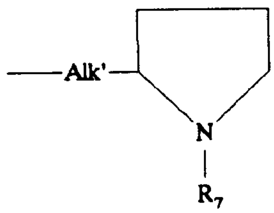

in which Alk' is a $C_1$-$C_3$ alkylene group and $R_7$ is a $C_1$-$C_4$ alkyl group; or

- a group

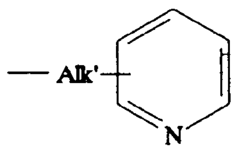

in which Alk' is as defined above and substitutes the pyridine in the 2-, 3- or 4-position;

with the limitation that $R_1$ and $R_2$ are not simultaneously hydrogen when $R_4$ is a group $(CH_2)_2NR_5R_6$; as well as the salts thereof with mineral or organic acids.

2. Compound according to claim 1 in which Ar is a group

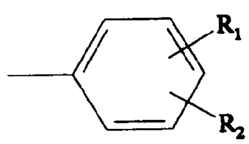

in which $R_1$ and $R_2$ are as defined in claim 1.

3. Compound according to one of claims 1 or 2, having the formula

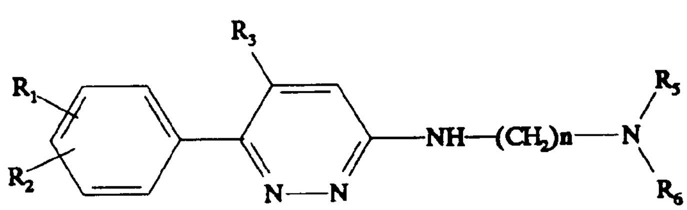

in which $R_1$, $R_2$, $R_3$, $R_5$ and $R_6$ are as defined in claim 1 and n is between 1 and 6, with the limitation that $R_1$ and $R_2$ are not simultaneously hydrogen when n = 2.

4. Compound according to claim 3, characterized in that n is equal to 2, 3 or 4.

**5.** Compound according to one of claims 1 or 2, having the formula

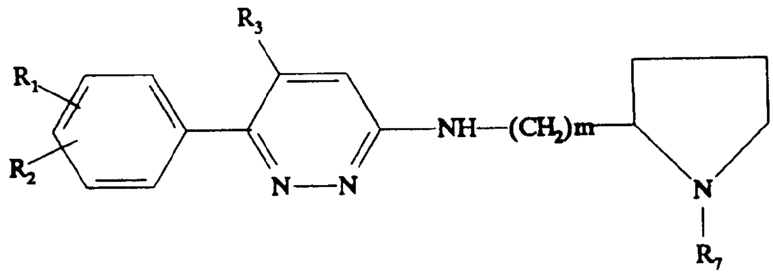

in which $R_1$, $R_2$, $R_3$ and $R_7$ are as defined in claim 1 and m is an integer between 1 and 3.

**6.** Compound according to claim 5, characterized in that m is equal to 1 or 2.

**7.** Method of preparing compounds of formula (I) according to claim 1, characterized in that

- an appropriately substituted ketone of the formula

$$Ar\text{-}COCH_2\text{-}R_3 \qquad \underline{1}$$

  is heated with ethyl glyoxylate to form the hydroxyester of the formula

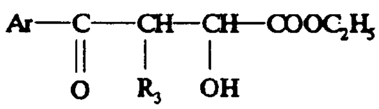

2

- the ester 2 is heated with hydrazine hydrate to form the hydroxypyridazinone of the formula

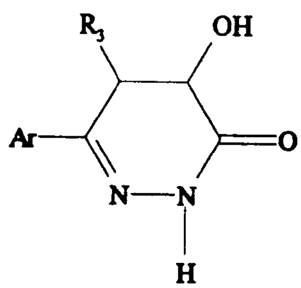

3

- the resulting product is dehydrated by heating in an acid medium, to form the 2H-pyridazin-3-one of the formula

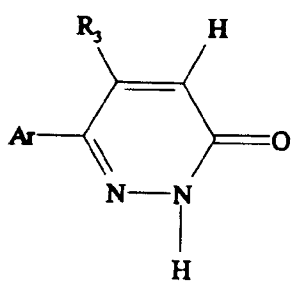

4

- the resulting product is treated with excess phosphorus oxychloride, under the action of heat, to form the 3-chloropyridazine of the formula

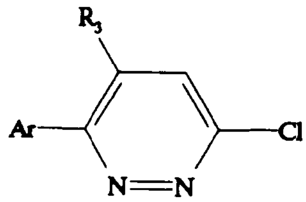

5

- the chlorine derivative 5 is heated with an excess of amine $R_4NH_2$, if appropriate in the presence of ammonium chloride, to give the compound (I);
- if appropriate, the compound (I) is converted to a salt with a mineral or organic acid by a known method.

**8.** Method according to claim 7, characterized in that a ketone 1 is used in which Ar is a group

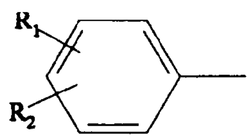

in which $R_1$ and $R_2$ are as defined in claim 1.

**9.** Method according to claim 7, characterized in that an amine $R_4NH_2$ is used in which $R_4$ is one of the following groups

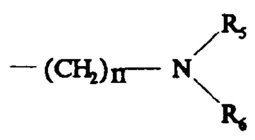

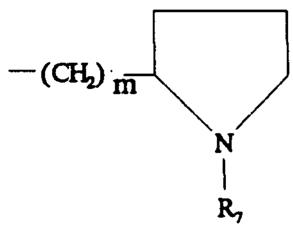

in which $R_5$, $R_6$, and $R_7$ are as defined in claim 1 and n is between 1 and 6, preferably equal to 2, 3 or 4 and m is between 1 and 3, preferably equal to 1 or 2.

**10.** Pharmaceutical compositions in which a compound of formula (I) or one of its pharmaceuticaly acceptable salts is present as the active principle, in combination with a pharmaceutically acceptable vehicle.

**11.** Pharmaceutical compositions according to claim 10, characterized in that they are in the form of unit dosages containing 5 to 200 mg of active principle.

**Claims for the following Contracting State : GR**

**1.** Pyridazine derivatives of the formula:

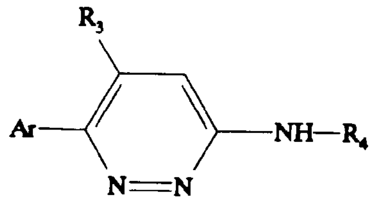 (I)

in which

- Ar is a group

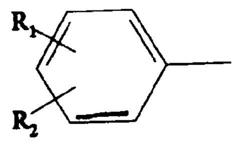,

a pyridyl or a thienyl;
- $R_1$ and $R_2$ independently are each hydrogen, a halogen atom, a hydroxyl group, a trifluoromethyl group, a $C_1$-$C_4$ alkoxy group or a $C_1$-$C_4$ alkyl group;
- $R_3$ is a $C_1$-$C_4$ alkyl group or a phenyl;
- $R_4$ is:
- a group

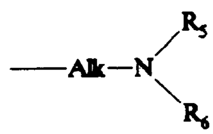

in which Alk is a $C_1$-$C_6$ alkylene group, $R_5$ is hydrogen or a $C_1$-$C_6$ alkyl group and $R_5$ is a $C_1$-$C_4$ alkyl group, a benzyl or a $C_3$-$C_7$ cycloalkyl, or else $R_5$ and $R_6$ form, with the nitrogen atom to which they are bonded, a heterocycle selected from morpholine, thiomorpholine, pyrrolidine, N-methylpiperazine and piperidine which is unsubstituted or substituted by one or more methyl groups, by a hydroxy, by a phenyl or by a benzyl;
- a group

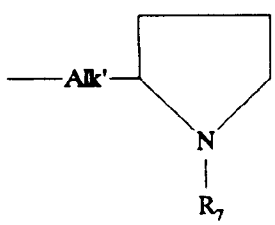

in which Alk' is a $C_1$-$C_3$ alkylene group and $R_7$ is a $C_1$-$C_4$ alkyl group; or
- a group

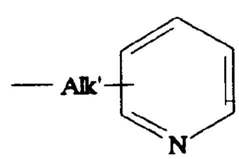

in which Alk' is as defined above and substitutes the pyridine in the 2-, 3- or 4-position;
with the limitation that $R_1$ and $R_2$ are not simultaneously hydrogen when $R_4$ is a group $(CH_2)_2NR_5R_5$;

as well as the salts thereof with mineral or organic acids.

2. Compound according to claim 1 in which Ar is a group

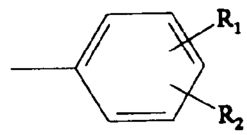

in which $R_1$ and $R_2$ are as defined in claim 1.

3. Compound according to one of claims 1 or 2, having the formula

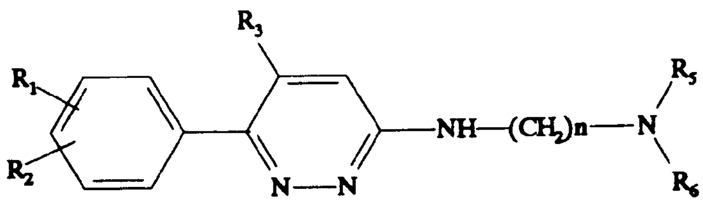

in which $R_1$, $R_2$, $R_3$, $R_5$ and $R_6$ are as defined in claim 1 and n is between 1 and 6, with the limitation that $R_1$ and $R_2$ are not simultaneously hydrogen when n = 2.

4. Compound according to claim 3, characterized in that n is equal to 2, 3 or 4.

5. Compound according to one of claims 1 or 2, having the formula

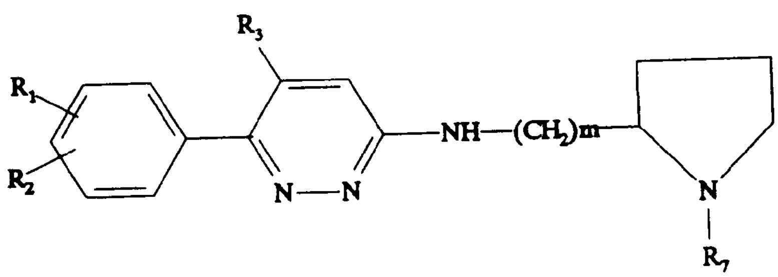

in which $R_1$, $R_2$, $R_3$ and $R_7$ are as defined in claim 1 and m is an integer between 1 and 3.

6. Compound according to claim 5, characterized in that m is equal to 1 or 2.

7. Method of preparing compounds of formula (I) according to claim 1, characterized in that:
   - an appropriately substituted ketone of the formula

     $Ar\text{-}COCH_2\text{-}R3$   1

     is heated with ethyl glyoxylate to form the hydroxyester of the formula

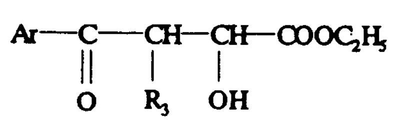

2

   - the ester 2 is heated with hydrazine hydrate to form the hydroxypyridazinone of the formula

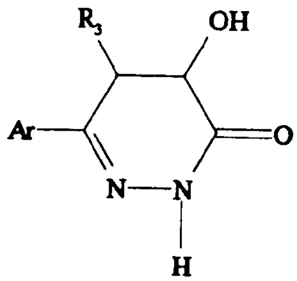

3

- the resulting product is dehydrated by heating in an acid medium, to form the 2H-pyridazin-3-one of the formula

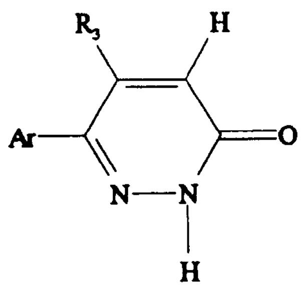

4

- the resulting product is treated with excess phosphorus oxychloride, under the action of heat, to form the 3-chloropyridazine of the formula

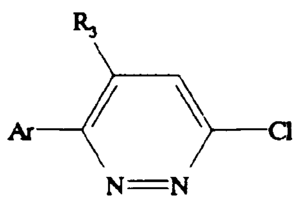

5

- the chlorine derivative 5 is heated with an excess of amine $R_4NH_2$, if appropriate in the presence of ammonium chloride, to give the compound (I);
- if appropriate, the compound (I) is converted to a salt with a mineral or organic acid by a known method.

8. Method according to claim 7, characterized in that a ketone 1 is used in which Ar is a group

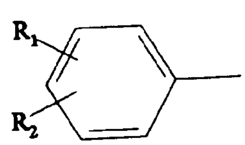

in which $R_1$ and $R_2$ are as defined in claim 1.

9. Method according to claim 7, characterized in that an amine $R_4NH_2$ is used in which $R_4$ is one of the following groups

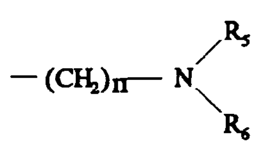

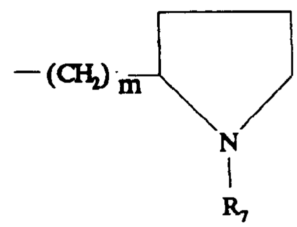

in which $R_5$, $R_6$, and $R_7$ are as defined in claim 1 and n is between 1 and 6, preferably equal to 2, 3 or 4 and m is between 1 and 3, preferably equal to 1 or 2.

10. Method of preparation of pharmaceutial compositions useful especially for the treatment of cholinergic deficiency characterized in that it consists in using as the active principle a compound of formula (I) as defined in any one of claims 1 to 6.

11. Method according to claim 10, characterized in that 5 to 200 mg of active principle are used to form compositions in the form of unit dosages.

**Claims for the following Contracting State : ES**

1. Method of preparation of pyridazine derivatives of the formula:

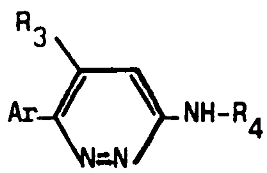

(I)

in which

- Ar is a group

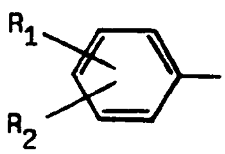

a pyridyl or a thienyl;

- $R_1$ and $R_2$ independently are each hydrogen, a halogen atom, a hydroxyl group, a trifluoromethyl group, a $C_1$-$C_4$ alkoxy group or a $C_1$-$C_4$ alkyl group;
- $R_3$ is a $C_1$-$C_4$ alkyl group or a phenyl;
- $R_4$ is:
- a group

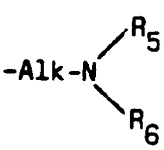

in which Alk is a $C_1$-$C_6$ alkylene group, $R_6$ is hydrogen or a $C_1$-$C_6$ alkyl group and $R_6$ is a $C_1$-$C_4$ alkyl group, a benzyl or a $C_3$-$C_7$ cycloalkyl, or else $R_6$ and $R_6$ form, with the nitrogen atom to which they are bonded, a heterocycle selected from morpholine, thiomorpholine, pyrrolidine, N-methylpiperazine and piperidine which is unsubstituted or substituted by one or more methyl groups, by a hydroxy, by a phenyl or by a benzyl;
- a group

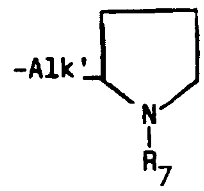

in which Alk' is a $C_1$-$C_3$ alkylene group and $R_7$ is a $C_1$-$C_4$ alkyl group; or

- a group

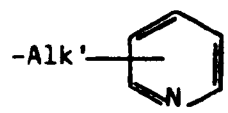

in which Alk' is as defined above and substitutes the pyridine in the 2-, 3- or 4-position;
with the limitation that $R_1$ and $R_2$ are not simultaneously hydrogen when $R_4$ is a group $(CH_2)_2NR_5R_6$;
as well as the salts thereof with mineral or organic acids;
characterized in that it consists in

- heating an appropriately substituted ketone of the formula

$Ar$-$COCH_2$-$R3$ 1

with ethyl glyoxylate to form the hydroxyester of the formula

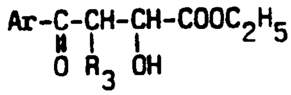

2

- heating the ester 2 with hydrazine hydrate to form the hydroxypyridazinone of the formula

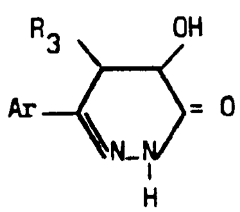

3

- dehydrating the resulting product, by heating in an acid medium, to form the 2H-pyridazin-3-one of the formula

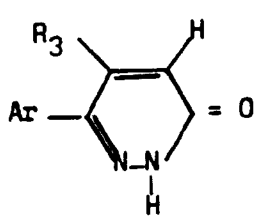

4

- treating the resulting product with excess phosphorus oxychloride, under the action of heat, to form the 3-chloropyridazine of the formula

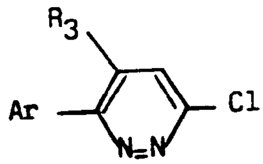

5

- heating the chlorine derivative 5 with an excess of amine $R_4NH_2$, if appropriate in the presence of ammonium chloride, to give the compound (I);
- if appropriate, converting, the compound (I) to a salt with a mineral or organic acid by a known method.

**2.** Method according to claim 1 characterized in that a ketone 1 is used in which Ar is a group

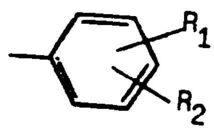

in which $R_1$ and $R_2$ are as defined in claim 1.

**3.** Method according to claim 1, characterized in that an amine $R_4NH_2$ is used in which $R_4$ is one of the following groups

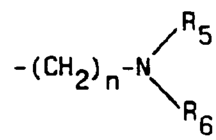

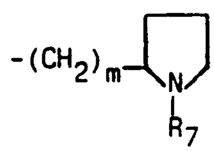

in which $R_5$, $R_6$, and $R_7$ are as defined in claim 1 and n is between 1 and 6, preferably equal to 2, 3 or 4 and m is between 1 and 3, preferably equal to 1 or 2.

**4.** Method of preparation of pharmaceutical compositions useful especially for the treatment of cholinergic deficiency characterized in that it consists in using as the active principle a compound of formula (I) as defined in any one of claims 1 to 3.

**5.** Method according to claim 4, characterized in that 5 to 200 mg of active principle are used to form compositions in the form of unit dosages.